# EUROPEAN PATENT SPECIFICATION

(11) **EP 0 720 981 B1**
(45) Date of publication and mention of the grant of the patent: **14.10.1998**
(21) Application number: 96100116.1
(22) Date of filing: 05.01.1996
(51) Int. Cl.: C07C 243/28, C07D 249/08, C07D 487/04, G03C 7/38

(54) **Cyclohexyloxycarbonylacetohydrazides and method for producing 1H-1,2,4-triazoles using the hydrazides**
Cyclohexyloxycarbonylacetohydrazide, und ihre Verwendung für die Herstellung von 1H-1,2,4-Triazolen
Cyclohexyloxycarbonylacétohydrazides et leur utilisation pour la préparation des 1H-1,2,4-triazoles

(30) Priority: 05.01.1995 JP 15481/95
(43) Date of publication of application: 10.07.1996
(73) Proprietor: FUJI PHOTO FILM CO., LTD., Kanagawa-ken (JP)
(72) Inventor: Ito, Takayuki, c/o Fuji Photo Film Co., Ltd., Minami-ashigara-shi, Kanagawa-ken (JP); Shimada, Yasuhiro, c/o Fuji Photo Film Co., Ltd., Minami-ashigara-shi, Kanagawa-ken (JP); Katsumata, Taiji, c/o Fuji Photo Film Co., Ltd., Minami-ashigara-shi, Kanagawa-ken (JP)
(74) Representative: Hansen, Bernd, Dr. Dipl.-Chem.

(56) References cited:
- EP-A- 0 491 197
- EP-A- 0 518 238
- EP-A- 0 628 867
- JOURNAL OF THE CHEMICAL SOCIETY, December 1962 LONDON, GB, pages 5149-52, E. J. BROWNE ET. AL. 'Triazoles. Part VII. Synthesis of Substituted 1,2,4-Triazoles.'

## Description

The present invention relates to novel hydrazide derivatives. The present invention also relates to a method for producing 1H-1,2,4-triazole derivatives -- which are important intermediates in the synthesis of 1H-pyrrolo-[1,2-b][1,2,4]triazole derivatives, which latter are useful as synthetic intermediates of physiologically active substances, such as medicines and pesticides, and which latter are also useful as photographic couplers or dyes -- by using said hydrazide derivatives.

### BACKGROUND OF THE INVENTION

1H-pyrrolo-[1,2-b][1,2,4]triazole derivatives are useful as synthetic intermediates of physiologically active substances, such as medicines and pesticides, and as photographic couplers or dyes. It is described in U.S. Patent No. 5,256,526, JP-A ("JP-A" means unexamined published Japanese patent application) Nos. 202049/1993, 204107/1993, and 172357/1994, and European Patent No. 545,300, that particularly 1H-pyrrolo-[1,2-b][1,2,4]triazole derivatives having an electron-attracting group in the 6- or 7-position are useful as photographic cyan couplers. Further, European Patent No. 628,867 describes 1H-pyrrolo-[1,2-b][1,2,4]triazole derivatives having, in the 7-position, for example, a cyclohexyloxycarbonyl group represented by the following formula (V): 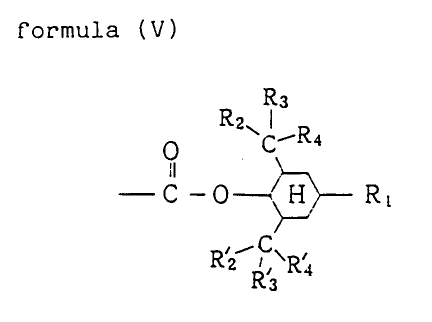 wherein R₁ represents a hydrogen atom or an alkyl group; R₂, R₃, R₄, R₂', R₃', and R₄', which are the same or different, each represent an alkyl group; and R₂ and R₃, and R₂' and R₃' each may bond together to form a ring.

On the other hand, for example, JP-A Nos. 255333/1993 and 202004/1993 describe methods for synthesizing 1H-pyrrolo-[1,2-b][1,2,4]triazole derivatives having an electron-attracting group in the 6- or 7-position. According to these methods, 1H-pyrrolo-[1,2-b][1,2,4]triazole derivatives having, in the 7-position, a cyclohexyloxycarbonyl group represented by formula (V), can be synthesized through 1H-1,2,4-triazole derivatives represented by the following formula (III): 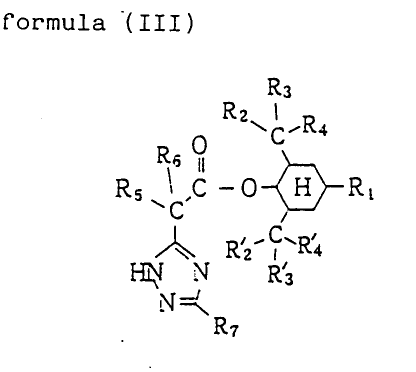 wherein R₁, R₂, R₃, R₄, R₂', R₃', and R₄' have the same meanings as defined above; R₅ and R_{6,} which are the same or different, each represent a hydrogen atom, a halogen atom, an alkyl group, or an aryl group; and R₇ represents an aliphatic group or an aryl group.

These 1H-1,2,4-triazole derivatives can be synthesized by methods described in the literature, for example, J. Chem. Soc., 1961, page 518, and ibid., 1962, page 5149; Angew. Chem., Vol. 72, page 956 (1960); Berichte., Vol. 97, page 3436 (1964); or in references cited therein, or by similar methods. For example, the 1H-1,2,4-triazole derivatives represented by formula (III) can be synthesized in accordance with the following scheme: 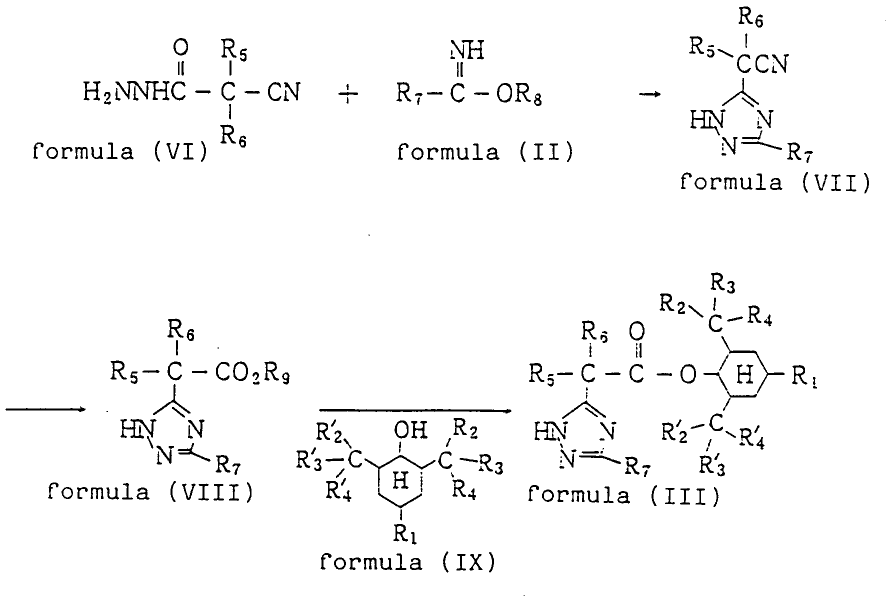 wherein R₁, R₂, R₃, R₄, R₂', R₃', R₄', R₅, R₆, and R₇ have the same meanings as defined above, R₈ represents a lower alkyl group, and R₉ represents a hydrogen atom or a lower alkyl group.

That is, the method comprises reacting cyanoacetohydrazide derivatives represented by formula (VI) with imido esters represented by formula (II), to form 1H-1,2,4-triazole derivatives represented by formula (VII), and then converting the cyano group to a desired cyclohexyloxycarbonyl group. However, it is difficult to carry out, in one step, the conversion of the cyano group to a desired cyclohexyloxycarbonyl group, and the conversion requires a step via an intermediate represented by formula (VIII). That is, to obtain the intended product efficiently, it is required that, after the cyano group is converted to a carboxyl group, condensation with an alcohol represented by formula (IX) is effected, or, after the cyano group is converted to a lower alkoxycarbonyl group, such as a methoxycarbonyl group or an ethoxycarbonyl group, transesterification with an alcohol represented by formula (IX) is effected. Therefore, the conventional method requires three steps to synthesize 1H-1,2,4-triazole derivatives represented by formula (III) from an imido ester represented by formula (II). Therefore it is desired to develop a simple method for synthesizing 1H-1,2,4-triazole derivatives represented by formula (III) in short steps.

### SUMMARY OF THE INVENTION

An object of the present invention is to provide a novel compound useful for producing, simply, a 1H-1,2,4-triazole derivative in short steps, which derivative is an important intermediate in the synthesis of a 1H-pyrrolo-[1,2-b][1,2,4]triazole derivative that is useful as a synthetic intermediate of a physiologically active substance, such as medicines and pesticides, and also useful as a photographic coupler and a dye.

Another object of the present invention is to provide a simple method for producing a 1H-1,2,4-triazole derivative in short steps by using the above compound.

Other and further objects, features, and advantages of the invention will appear more fully from the following description.

### DETAILED DESCRIPTION OF THE INVENTION

The present inventors, having keenly studied to attain the above objects, have found that the above objects can be attained by the following means:
(1) A cyclohexyloxycarbonylacetohydrazide compound represented by the following formula (I): 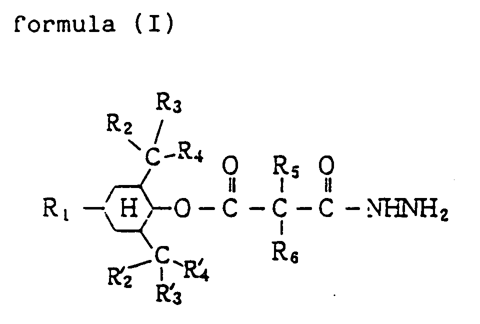 wherein R₁ represents a hydrogen atom or an alkyl group; R₂, R₃, R₄, R₂', R₃', and R₄', which are the same or different, each represent an alkyl group; R₂ and R₃, and R₂' and R₃' each may bond together to form a ring; and R₅ and R₆, which are the same or different, each represent a hydrogen atom, a halogen atom, an alkyl group, or an aryl group.
(2) A method for producing 1H-1,2,4-triazole represented by the following formula (III), comprising reacting a cyclohexyloxycarbonylacetohydrazide represented by formula (I), as stated in the above (1), with an imido ester represented by the following formula (II) (the reaction is represented by the scheme shown below): 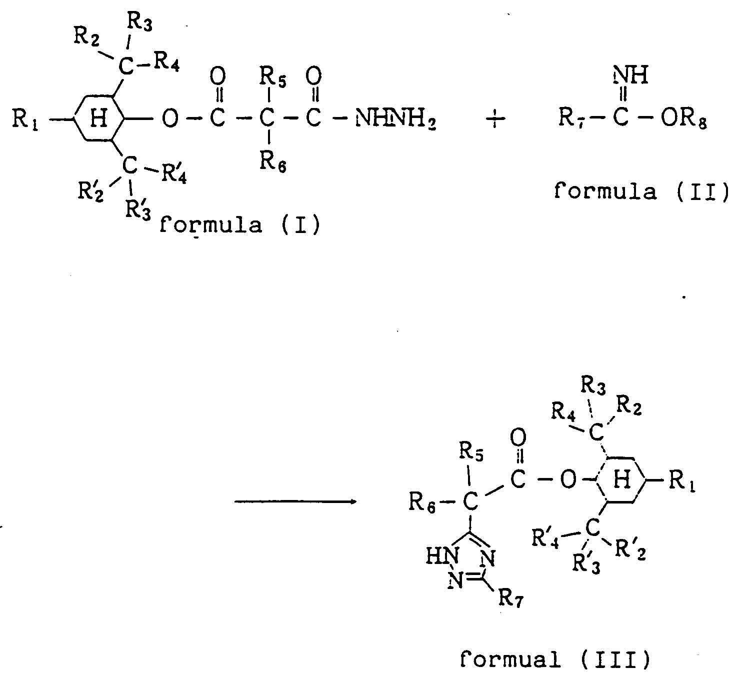 wherein R₁, R₂, R₃, R₄, R₂', R₃', R₄', R₅, and R₆ have the same meanings as defined above, R₇ represents an aliphatic group or an aryl group, and R₈ represents a lower alkyl group.
(3) The method as stated in the above (2), wherein the cyclohexyloxycarbonylacetohydrazide represented by formula (I) is subjected to condensation reaction with the imido ester represented by formula (II), to give an intermediate represented by the following formula (IV), and then the intermediate is subjected to intramolecular dehydrogenation condensation, to give a 1H-1,2,4-triazole represented by formula (III) (the reaction is represented by the scheme shown below): 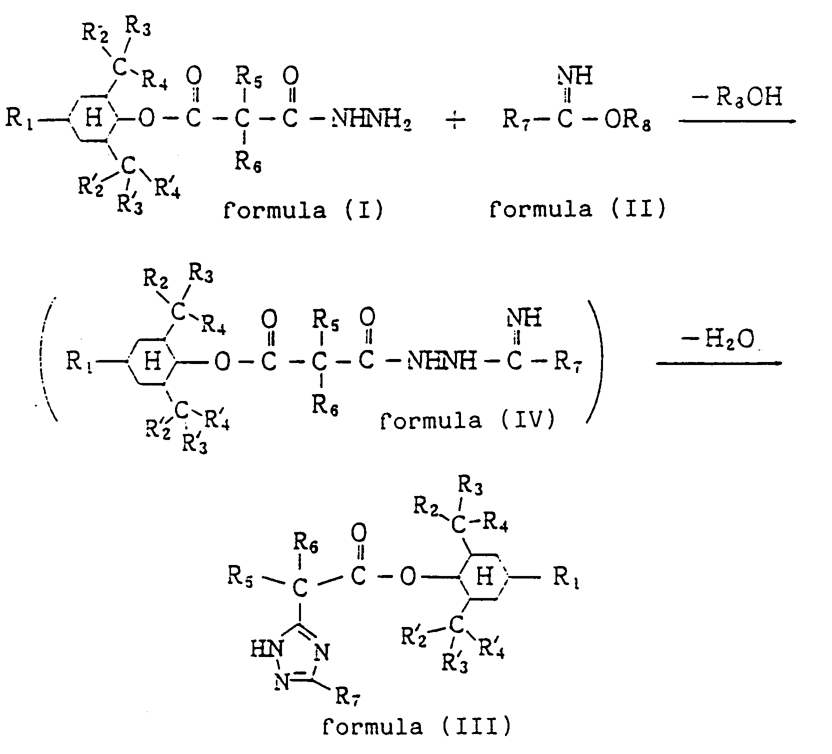 wherein R₁, R₂, R₃, R₄, R₂', R₃', R₄', R₅, R₆, R₇, and R₈ have the same meanings as defined above.

Now, the present invention is described in detail.

In formula (I), R₁ represents a hydrogen atom, or a straight-chain, branched-chain or cyclic alkyl group having 1 to 36 carbon atoms, such as methyl, ethyl, propyl, isopropyl, isobutyl, t-butyl, octyl, octadecyl, or cyclohexyl.

R₁ preferably represents a straight-chain, branched-chain or cyclic alkyl group having preferably 1 to 24 carbon atoms, and more preferably 1 to 12 carbon atoms, which may be substituted. Preferable substituents are a halogen atom, a hydroxyl group, a cyano group, a nitro group, a carboxyl group, an amino group, an alkoxy group, an aryloxy group, a heterocyclic oxy group, an acylamino group, an alkylamino group, an anilino group, a ureido group, a sulfamoylamino group, an alkylthio group, an arylthio group, a heterocyclic thio group, an alkoxycarbonylamino group, an aryloxycarbonylamino group, a sulfonamido group, a carbamoyl group, a sulfamoyl group, a sulfonyl group, an alkoxycarbonyl group, an aryloxycarbonyl group, an acyloxy group, a carbamoyloxy group, a silyloxy group, an imido group, a sulfinyl group, and a phosphonyl group. Particularly preferably, R₁ represents a methyl group.

R₂, R₃, R₄, R₂', R₃', and R₄', each represent a straight-chain, branched-chain or cyclic alkyl group having 1 to 24 carbon atoms.

R₂, R₃, R₄, R₂', R₃', and R₄, which are the same or different, each represent a straight-chain, branched-chain or cyclic alkyl group preferably having 1 to 12 carbon atoms, and more preferably 1 to 6 carbon atoms, such as methyl, ethyl, propyl, and cyclohexyl. R₂ and R₃, and R₂' and R₃' may bond together to form a ring (preferably a 6-membered ring, such as cyclohexyl), respectively. Particularly preferably, R₂, R₃, R₄, R₂', R₃', and R₄' each represent a methyl group.

R₅ and R₆, which are the same or different, each represent a hydrogen atom, a halogen atom (e.g., a fluorine atom, a chlorine atom, a bromine atom, and an iodine atom), an alkyl group [a straight-chain, branched-chain or cyclic alkyl group having 1 to 36 carbon atoms (preferably 1 to 24 carbon atoms), which may be substituted by such a substituent as described for R₁, e.g., methyl, ethyl, propyl, butyl, isopropyl, octyl, hexadecyl, cyclohexyl, and 1-cyano(methoxycarbonyl)methyl], or an aryl group [an aryl group having 6 to 36 carbon atoms (preferably 6 to 24 carbon atoms), which aryl group may be substituted by such a substituent as described for R₁, e.g., a phenyl group].

Preferably at least one of R₅ and R₆ represents a hydrogen atom, and more preferably both of R₅ and R₆ represent a hydrogen atom.

A more preferable embodiment of cyclohexyloxycarbonylacetohydrazides represented by formula (I) can be represented by the following formula (I'), and particularly preferably can be represented by the following formula (I''): 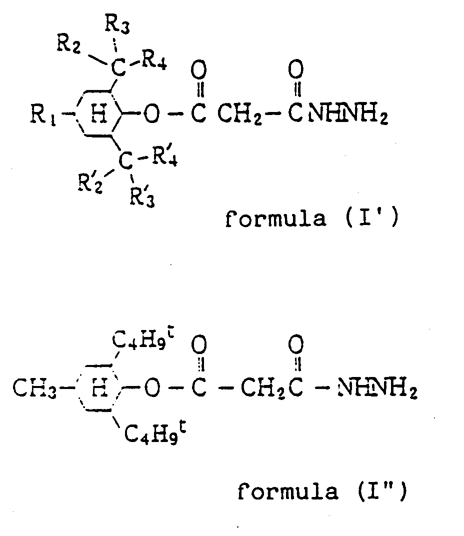 wherein R₁, R₂, R₃, R₄, R₂', R₃', and R₄' have the same meanings as defined above, and preferable ones of the groups represented by them are the same as those above.

Specific examples of cyclohexyloxycarbonylacetohydrazides represented by formula (I) of the present invention are shown below, but the present invention is not restricted to them.

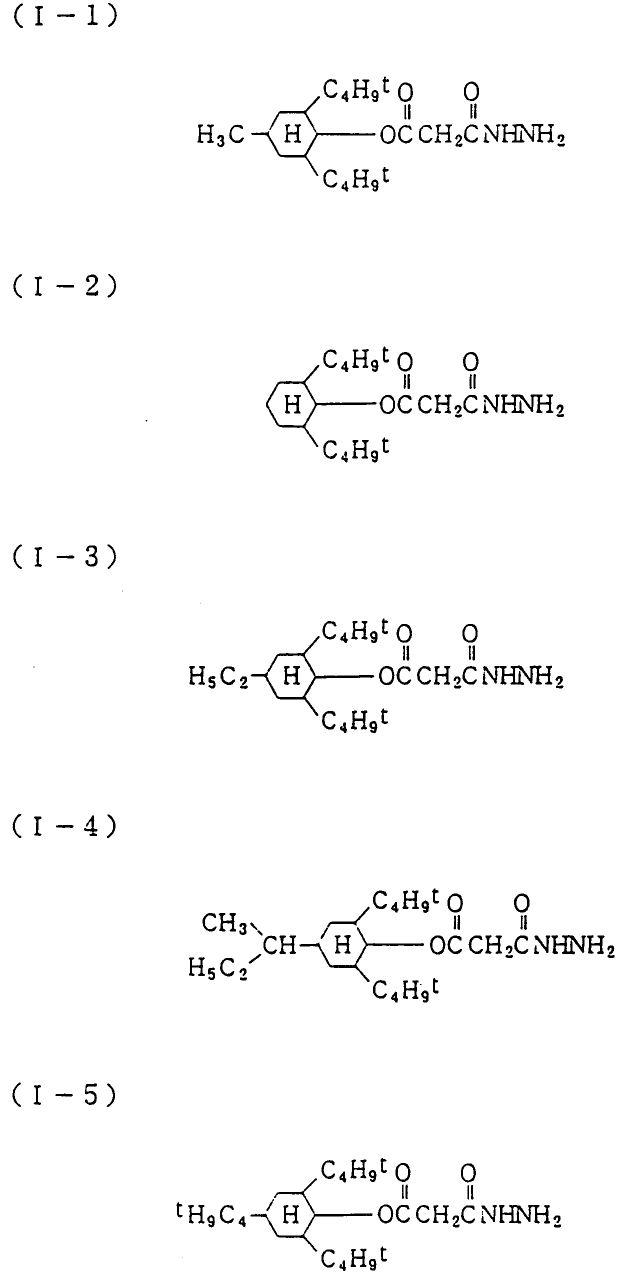

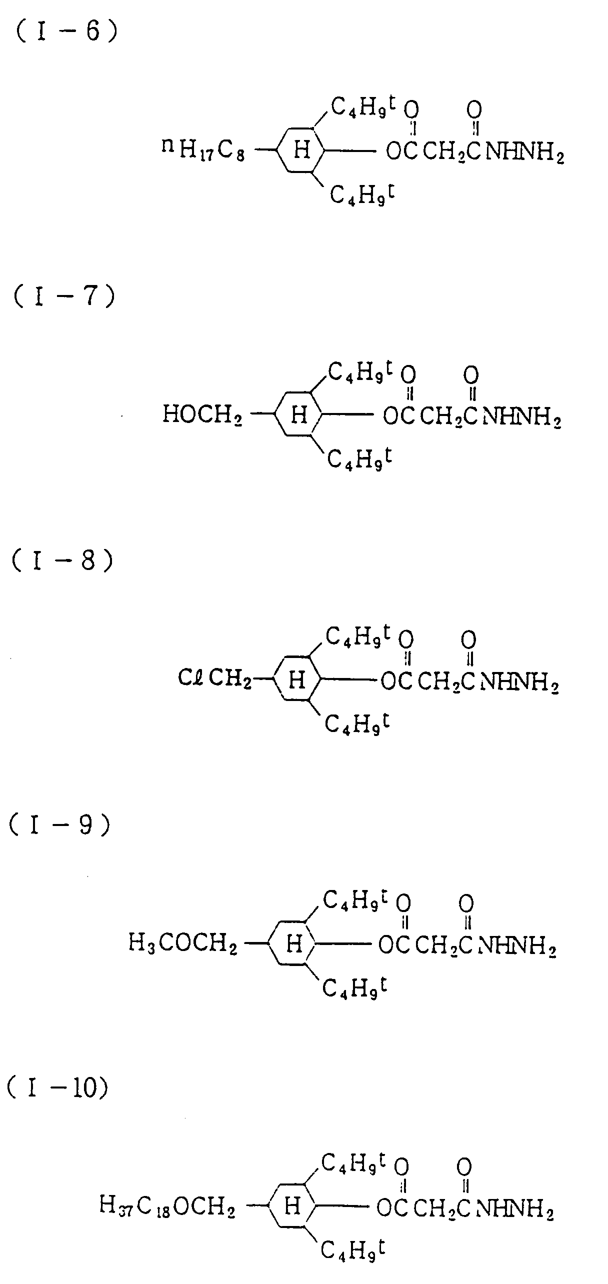

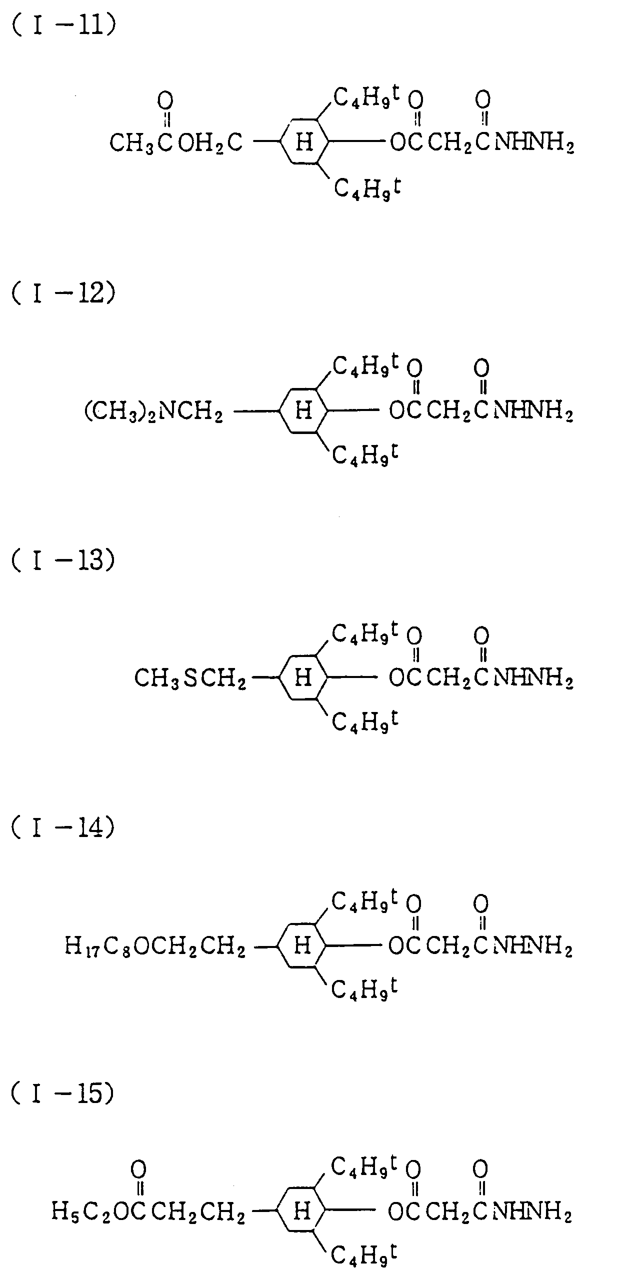

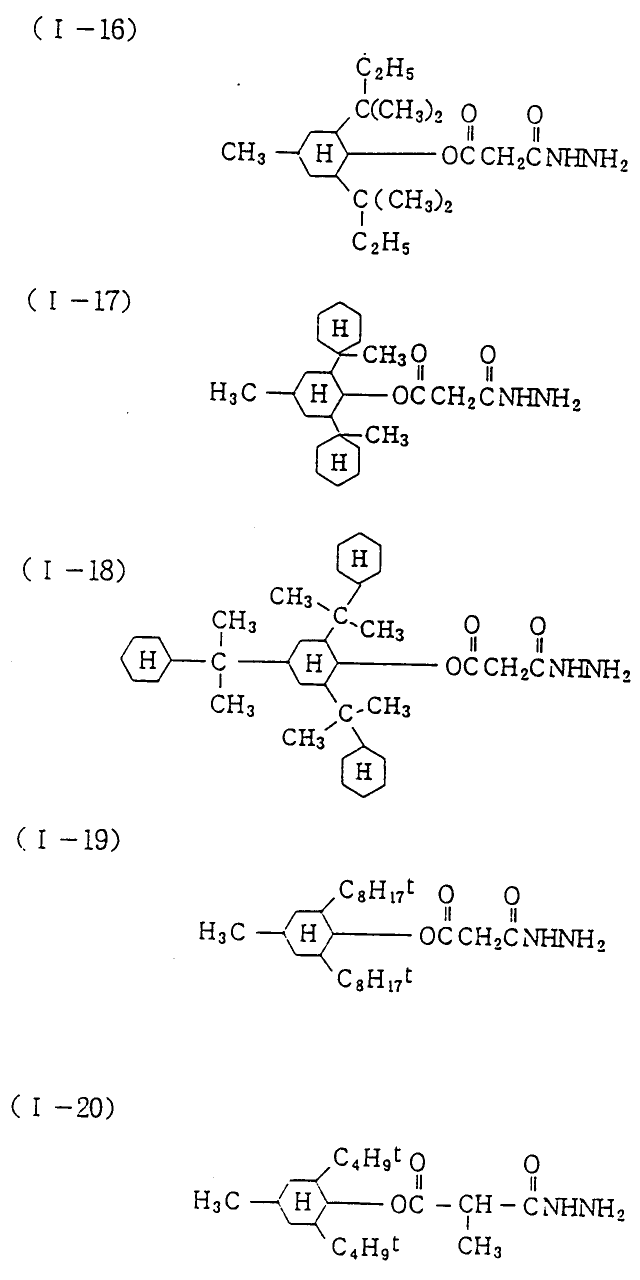

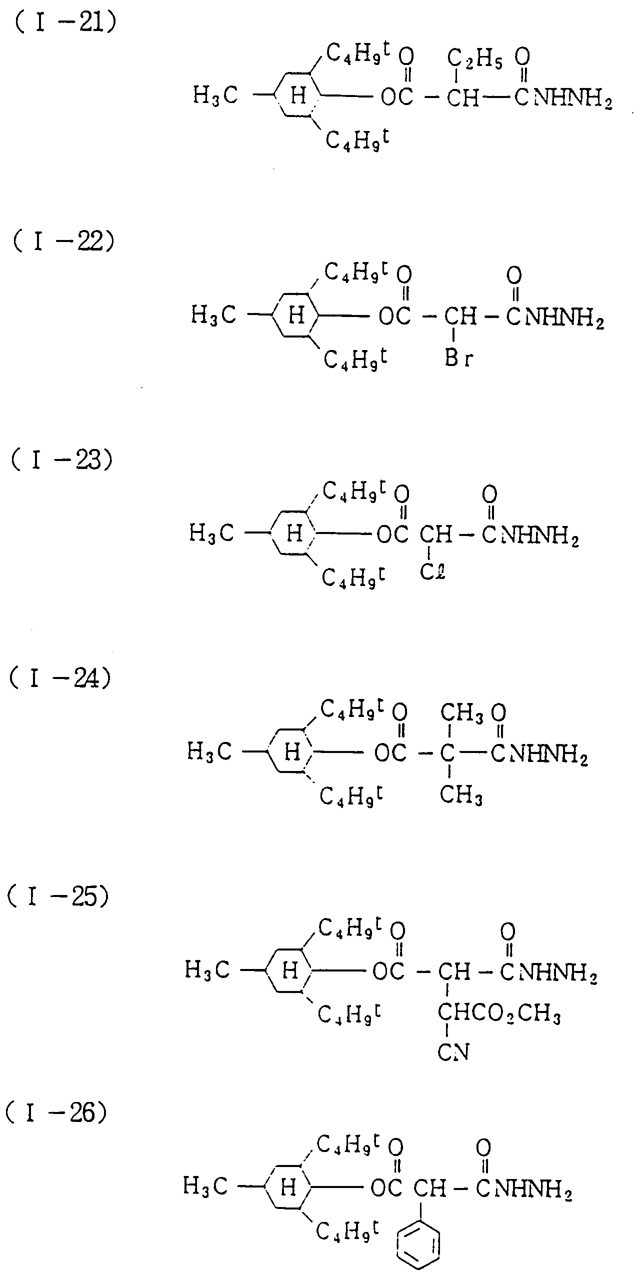

The method for synthesizing hydrazides represented by formula (I) is described now.

Hydrazides represented by formula (I) can be synthesized easily by condensing, for example, as shown in the following scheme, a half-ester derivative (a) of malonic acid, with an alcohol represented by (b), to produce (c) (hereinafter referred to as Step 1), and then letting hydrazine interact with (c) (hereinafter referred to as Step 2).

In this connection, the alcohols represented by (b) can be synthesized by catalytically hydrogenating corresponding phenols, by using a nickel catalyst, to produce cyclohexanones, and then reducing the cyclohexanones with aluminum lithium hydride (see J. Am. Chem. Soc., Vol. 79, 5019-5023 (1957)). 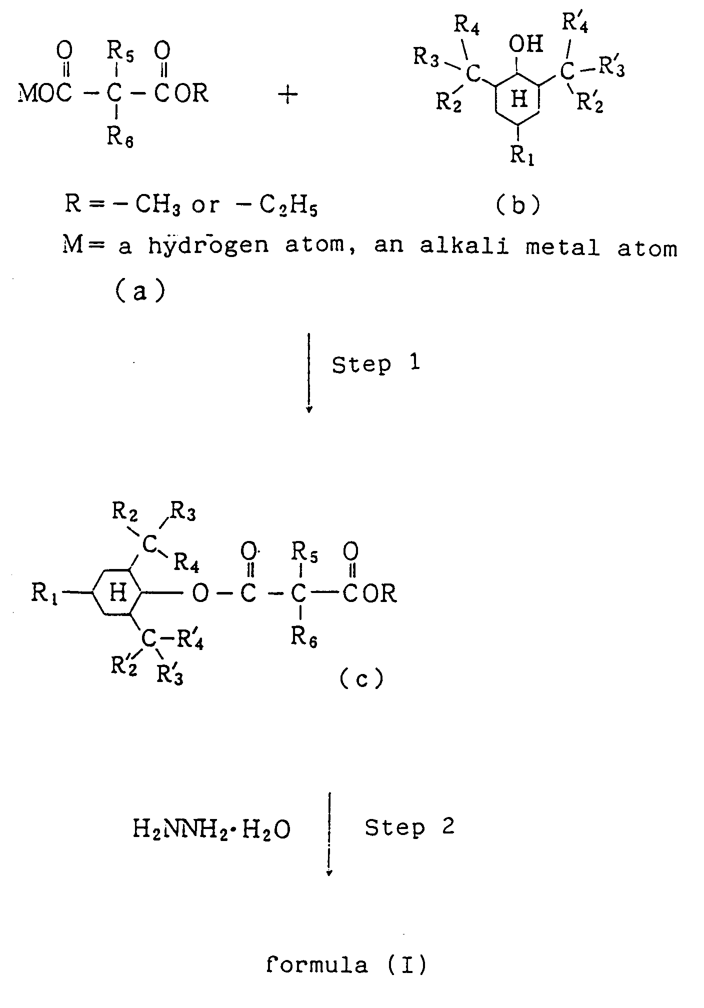 wherein R₁, R₂, R₃, R₄, R₂', R₃', R₄', R₅, and R₆ have the same meaning as defined above, R represents a methyl group or an ethyl group, and M represents a hydrogen atom or an alkali metal atom.

Step 1 is now described in detail.

The condensation in Step 1 can be attained by various general condensation methods for condensing carboxylic acids with alcohols (e.g., a method described in Jikken Kagaku-koza 22, Yukigosei IV, pages 43 to 50, 4th edition, edited by Nihonkagakukai, published by Maruzen Kabushiki Kaisha, and methods cited in the same reference), and, in particular, a particularly preferable result can be obtained by using trifluoroacetic anhydride as a condensation agent.

Reaction conditions under which a half-ester derivative (a) of malonic acid is condensed with an alcohol (b) in the presence of a condensation agent are described below.

The molar ratio of (a) to (b) is generally from 0.01 to 100, preferably from 0.1 to 10, and more preferably from 0.5 to 2.0.

The molar ratio of the condensation agent to (a) is generally from 0.01 to 1,000, preferably from 0.1 to 100, and more preferably from 0.5 to 10.

Example reaction solvents are methylene chloride, chloroform, 1,2-dichloroethane, acetonitrile, N,N-dimethylformamide, N,N-dimethylacetamide, dimethyl sulfoxide, dioxane, tetrahydrofuran, carbon tetrachloride, benzene, toluene, ethyl acetate, and the like.

The reaction temperature is generally -78 to 100°C, preferably -20 to 50°C, and more preferably -10 to 30°C.

The reaction time is generally 1 min to 72 hours, preferably 5 min to 36 hours, and more preferably 15 min to 24 hours.

Now, Step 2 is described in detail.

The molar ratio of the hydrazine hydrate to (c) is generally from 0.1 to 1,000, preferably from 0.5 to 100, and more preferably from 1 to 10.

Example reaction solvents are methanol, ethanol, isopropanol, t-butanol, t-amyl alcohol, ethylene glycol, ethylene glycol monomethyl ether, dioxane, tetrahydrofuran, dimethyl sulfoxide, and the like. The reaction can also be carried out without any solvent.

The reaction temperature is generally 0 to 200°C, preferably 20 to 150°C, and more preferably 50 to 100°C.

The reaction time is generally 1 min to 72 hours, preferably 15 min to 36 hours, and more preferably 30 min to 24 hours.

Now, formula (II) is explained.

R₇ represents an aliphatic group or an aryl group.

The aliphatic group represented by R₇ is a straight-chain alkyl group, a branched-chain alkyl group, an aralkyl group, an alkenyl group, an alkynyl group, a cycloalkyl group, and a cycloalkenyl group, having 1 to 36 (preferably 1 to 24) carbon atoms, any of which groups may have such substituents as described for R₁, for example, methyl, ethyl, propyl, isopropyl, t-butyl, t-amyl, octyl, octadecyl, vinyl, cyclohexyl, 4-pentylcyclohexyl, cyclohexenyl, and propargyl.

The aryl group represented by R₇ is an aryl group having 6 to 36 carbon atoms (preferably 6 to 24 carbon atoms), which group may have such substituents as described for R₁, for example, phenyl, 3-nitrophenyl, 4-nitrophenyl, 4-chlorophenyl, 3,5-dichlorophenyl, 4-methoxyphenyl, 4-t-butylphenyl, 3-(2-octoxy-5-t-octylphenylsulfonamido)-4-methoxyphenyl, and 3-nitro-4-methylphenyl.

R₈ represents a straight-chain, branched-chain or cyclic lower alkyl group having 1 to 8 carbon atoms (preferably 1 to 4 carbon atoms), such as methyl, ethyl, propyl, butyl, and octyl. Particularly preferably, R₈ represents a methyl group or an ethyl group.

Now, specific examples of imido esters represented by formula (II) according to the present invention are shown below, but the present invention is not limited to them.

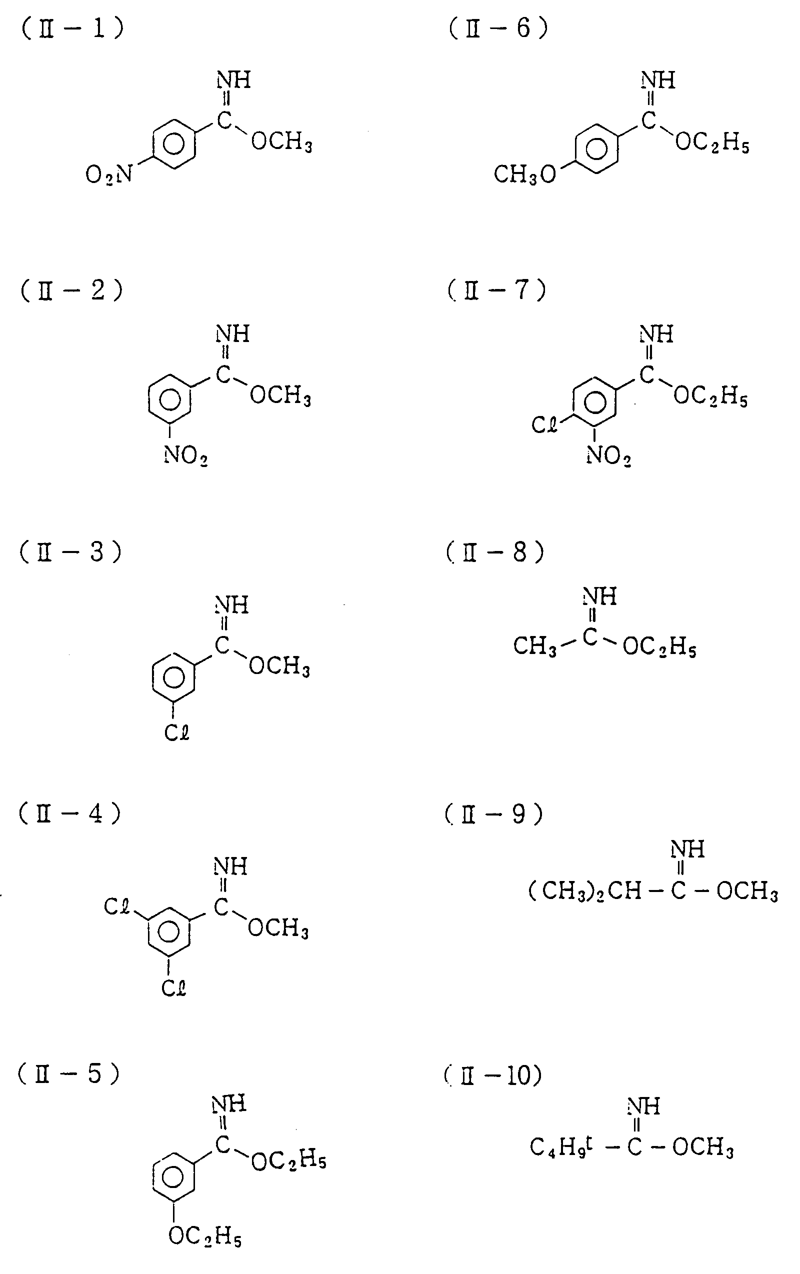

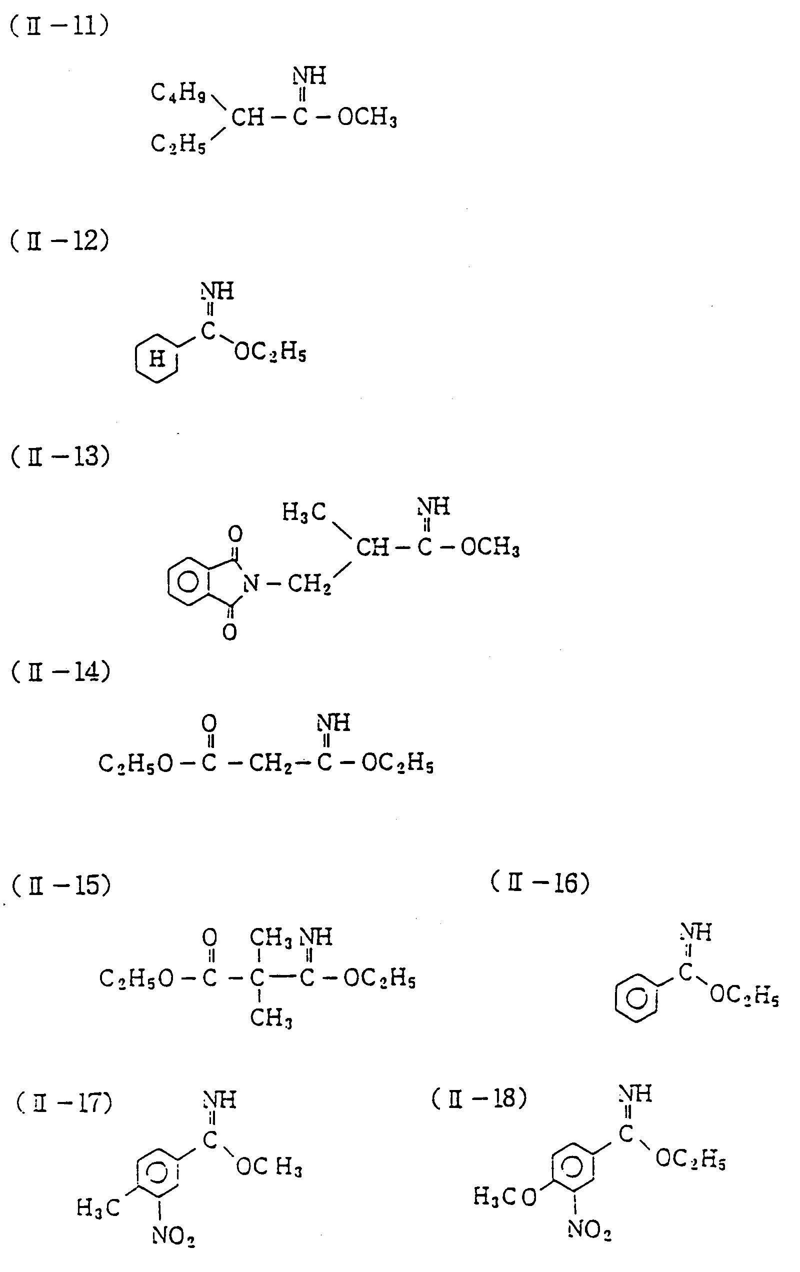

The imido esters represented by formula (II) can be synthesized by known methods; for example, by a method wherein a corresponding nitrile is subjected to addition of a lower alcohol in the presence of hydrochloric acid (Pinner method); and by a method wherein a corresponding nitrile is subjected to addition of a lower alcohol using a base as a catalyst (e.g., see Shin-jikken Kagaku-koza 14, Yukikagobutsu no Gosei to Hannou III, pages 1598 to 1608, edited by Nihonkagakukai, published by Maruzen Kabushiki Kaisha, and references cited therein).

Now, the method for producing triazoles represented by formula (III) by reacting hydrazides represented by formula (I) with imido esters represented by formula (II) is described.

The molar ratio of the imido esters represented by formula (II) to the hydrazides represented by formula (I) is generally from 0.1 to 10, preferably from 0.5 to 2, and more preferably 0.8 to 1.2.

Example reaction solvents are methanol, ethanol, isopropanol, t-amyl alcohol, ethylene glycol, ethylene glycol monomethyl ether, dioxane, tetrahydrofuran, dimethyl sulfoxide, N,N-dimethylformamide, N,N-dimethylacetamide, acetic acid, benzene, toluene, anisole, chlorobenzene, and the like.

The reaction can also be carried out without any solvent.

The reaction temperature is generally -20 to 200°C, preferably -10 to 180°C, and more preferably 0 to 150°C.

The reaction time is generally 1 min to 72 hours, preferably 15 min to 36 hours, and more preferably 30 min to 24 hours.

This reaction proceeds via the intermediate represented by formula (IV), which in turn undergoes dehydration condensation. Therefore, it is possible to change the above reaction conditions before and after the production of the intermediate, and in some cases it is possible to take out the intermediate. 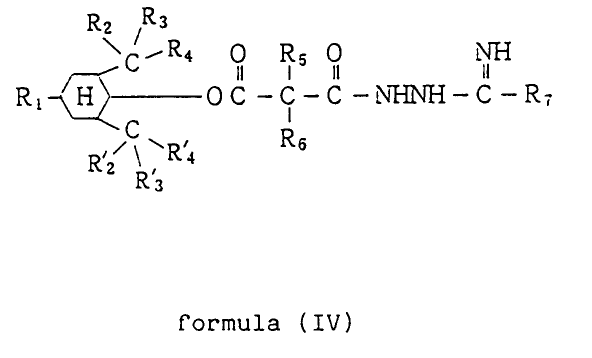 wherein R₁, R₂, R₃, R₄, R₂', R₃', R₄', R₅, R₆, and R₇ have the same meanings as defined above.

Specific examples of 1H-[1,2,4]-triazoles represented by formula (III) produced by the method of the present invention are shown below, but the present invention is not restricted to them.

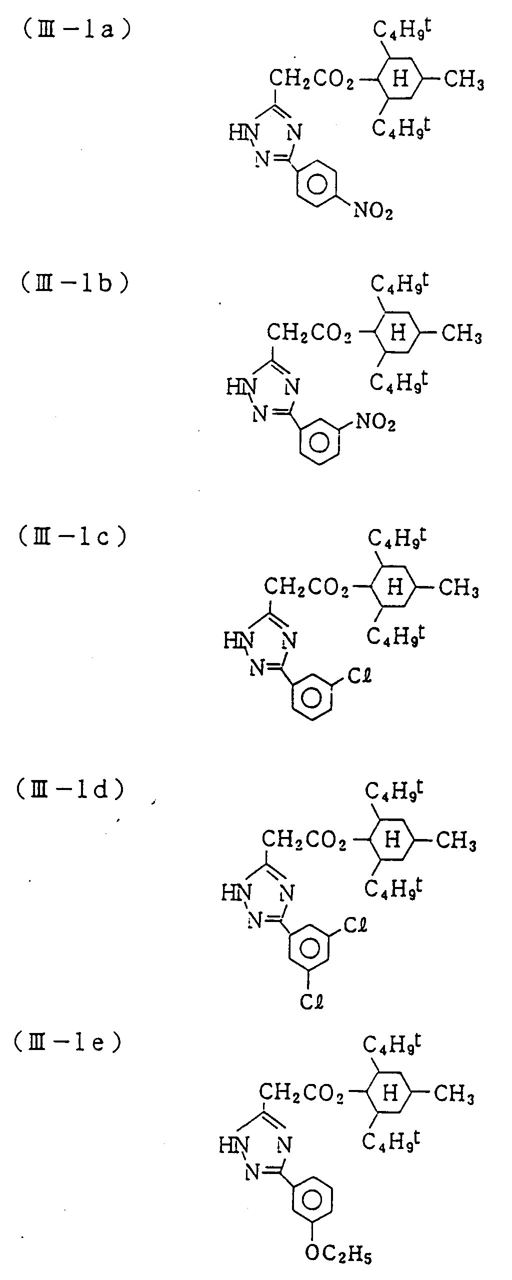

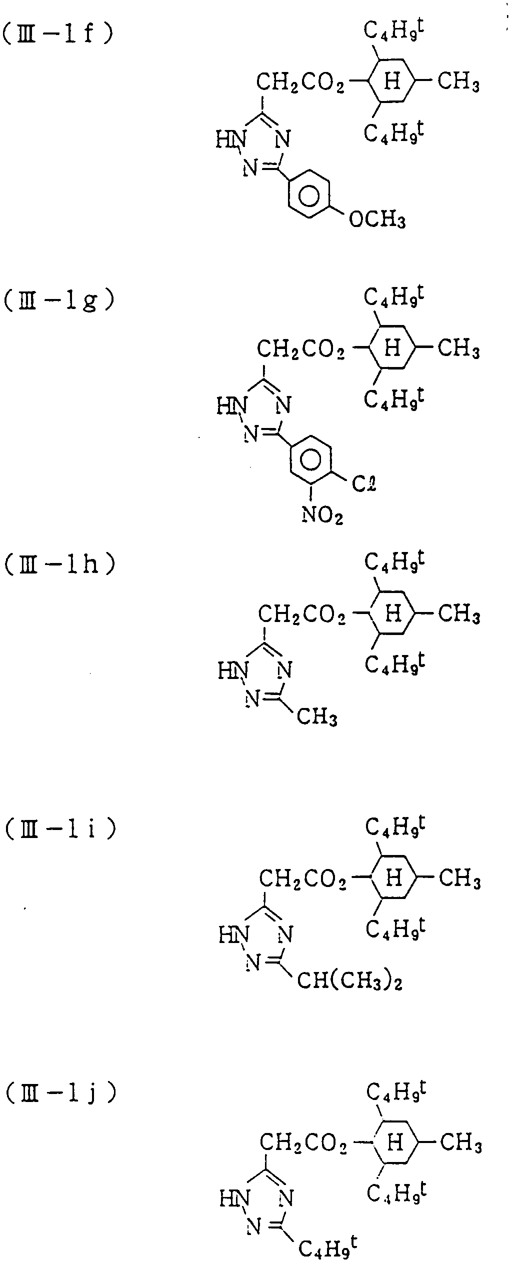

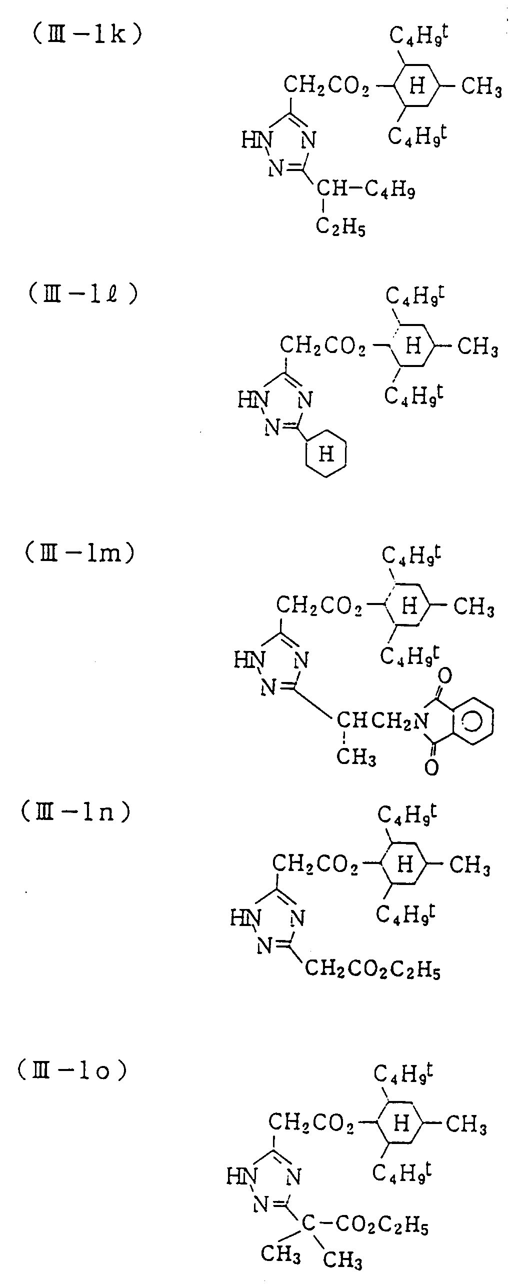

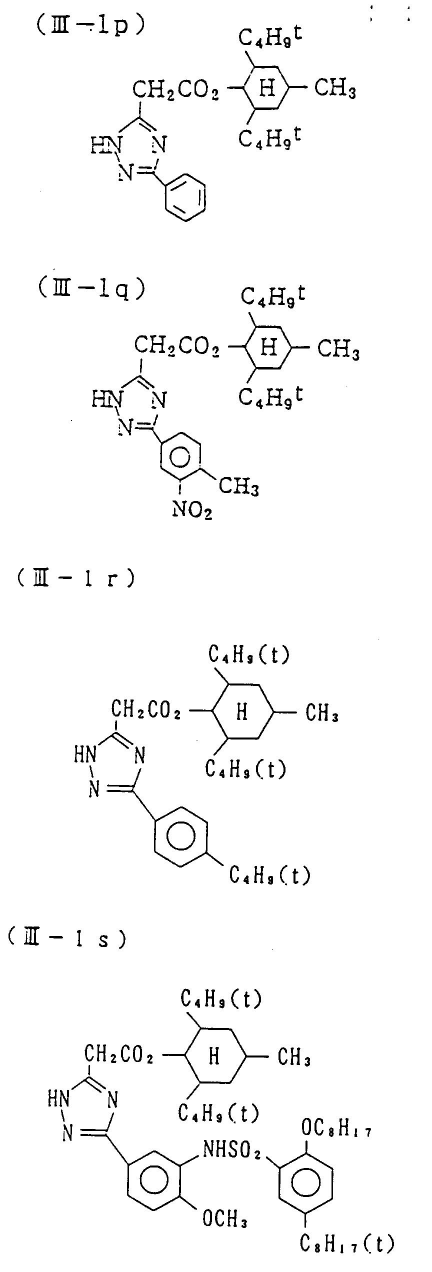

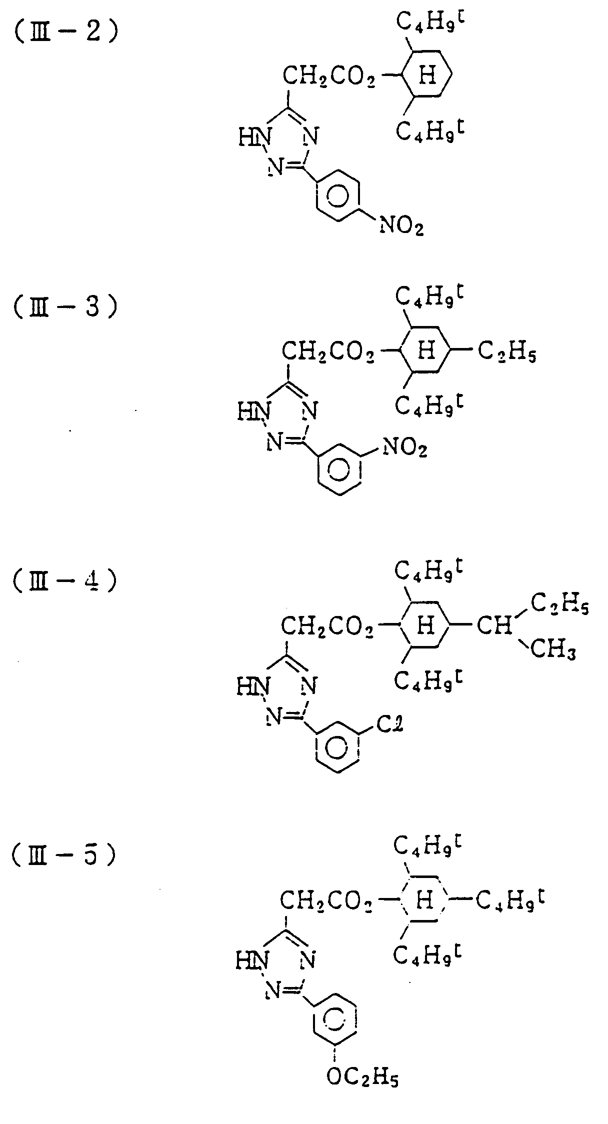

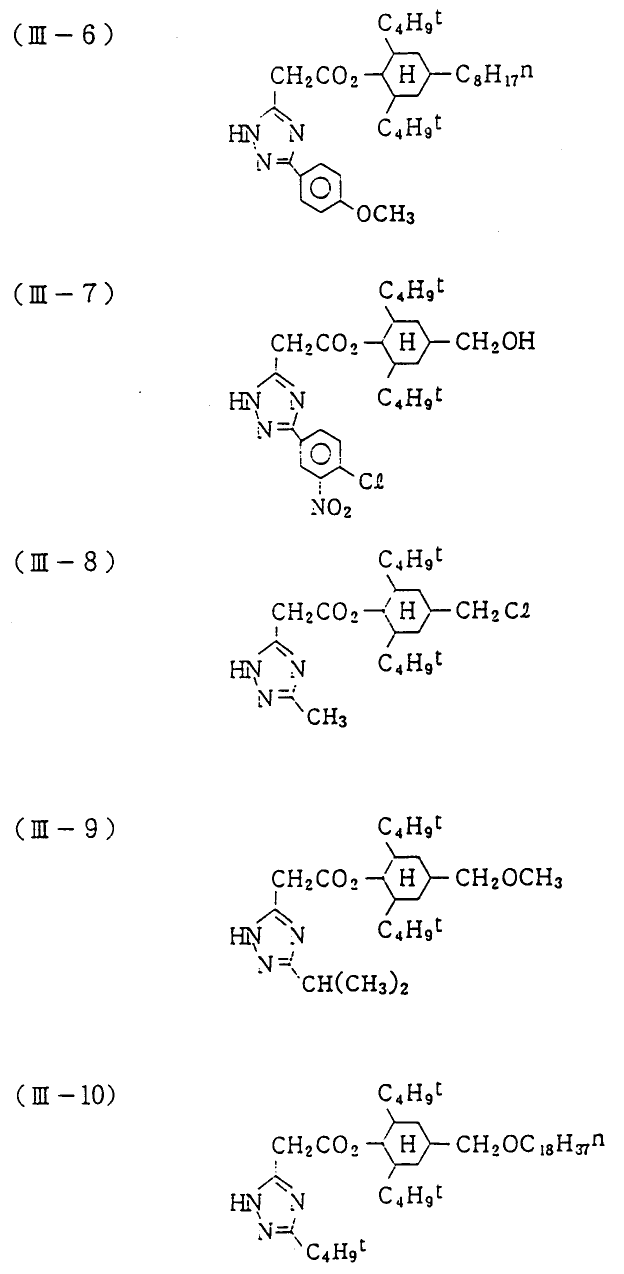

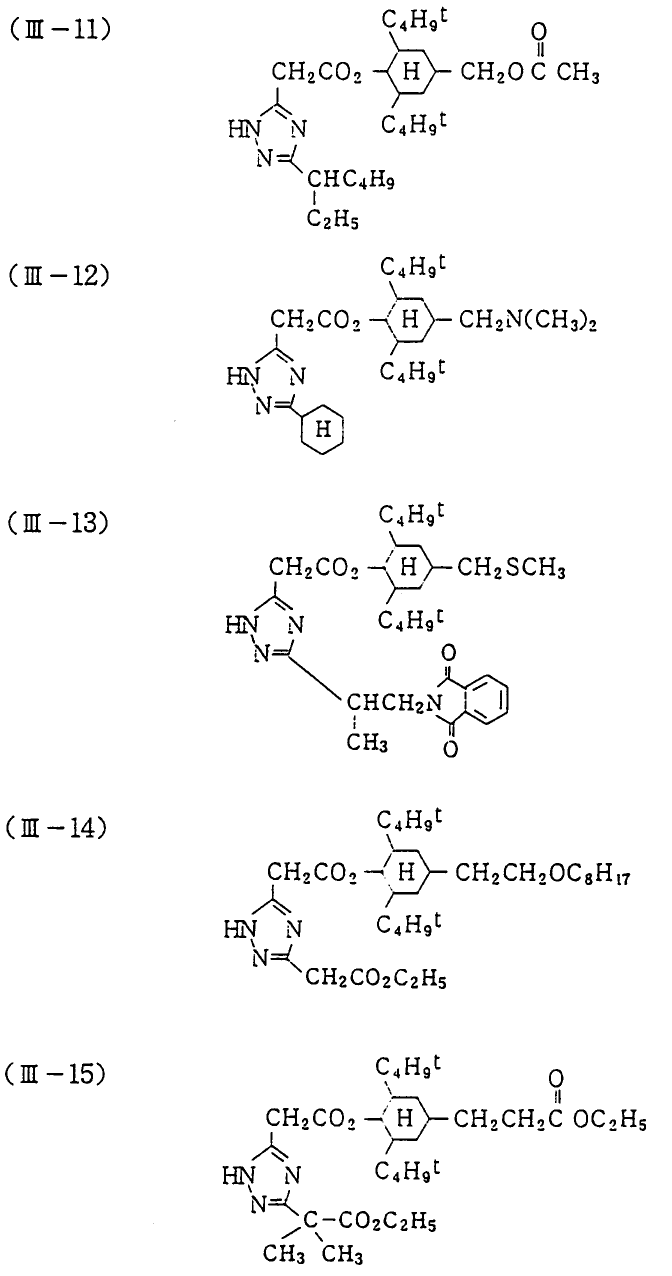

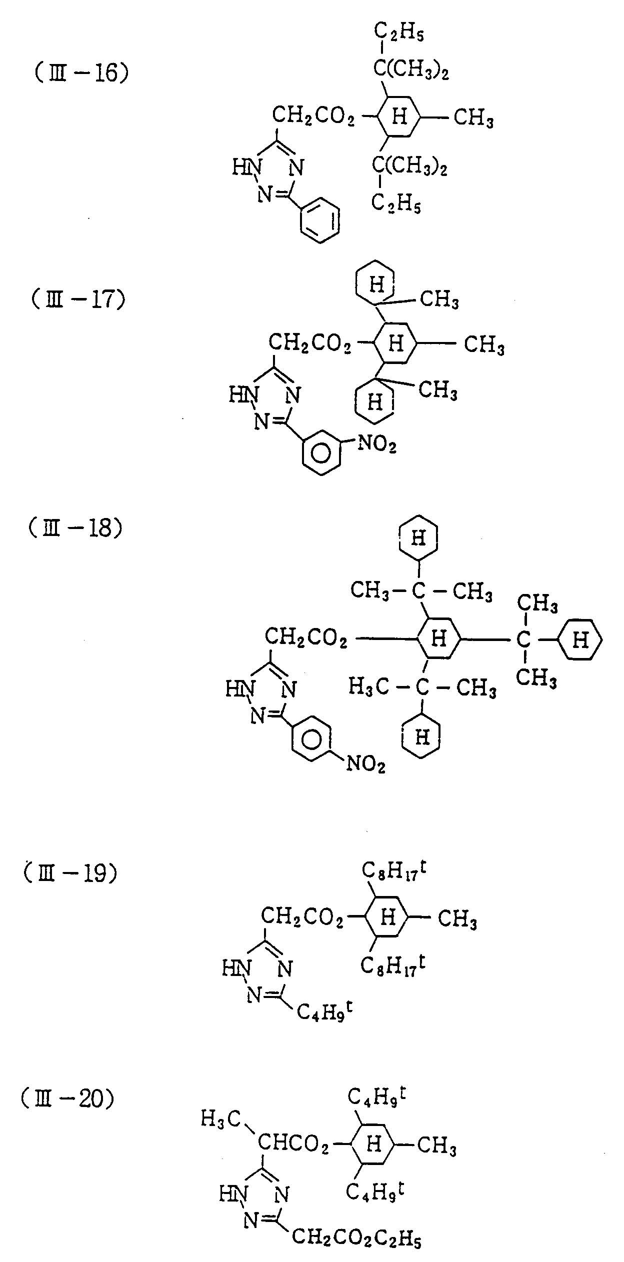

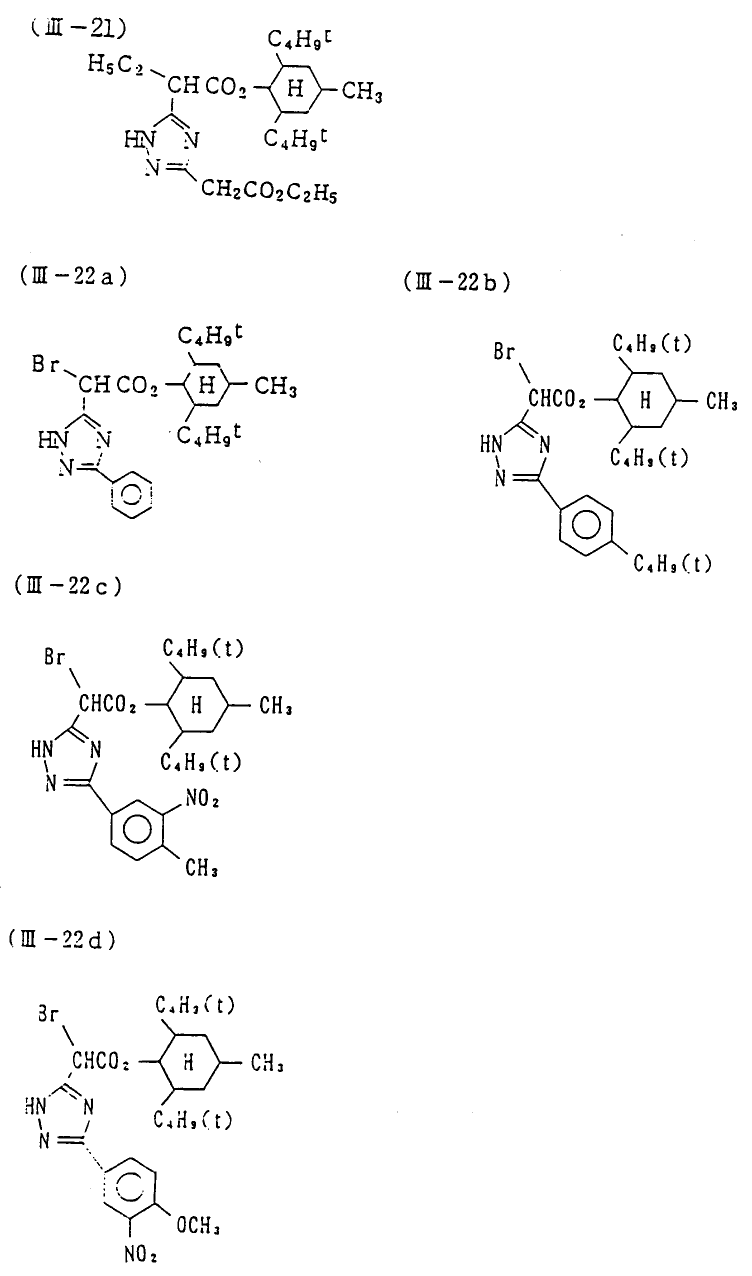

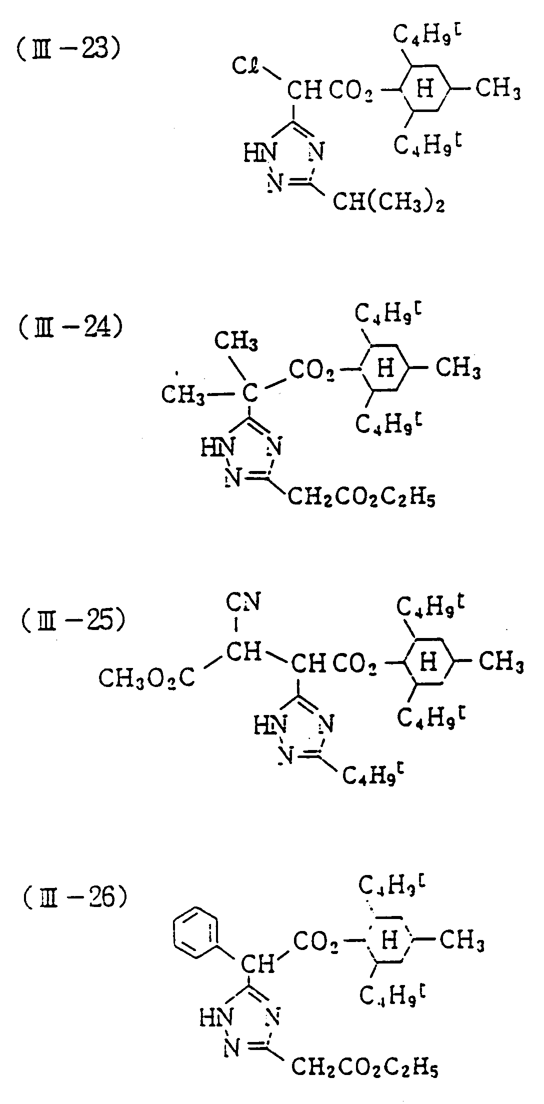

In the present invention, 1H-1,2,4-triazoles represented by formula (III) are obtained from the hydrazides represented by formula (I) and the imide esters represented by formula (II), via the intermediate represented by formula (IV), and the resultant triazoles of formula (III) can be converted to 1H-pyrrolo-[1,2-b][1,2,4]triazoles represented by the following formula (X): 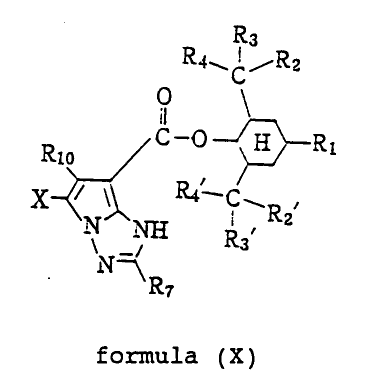 wherein R₁, R₂, R₃, R₄, R₂', R₃', R₄', and R₇ each have the same meanings as defined above, and the preferable ranges for these are the same as defined above; R₁₀ represents a hydrogen atom or a substituent, preferably R₁₀ represents an electron-withdrawing group (having the Hammett's substituent constant ap of 0.2 or more), such as a cyano group, an alkoxycarbonyl group (e.g., methoxycarbonyl, ethoxycarbonyl), and a perfluoroalkyl group (e.g., trifluoromethyl, heptafluoropropyl); and X represents a hydrogen atom or a substituent, preferably X represents a hydrogen atom, a carbamoyloxy group (e.g., N,N-diethylcarbamoyloxy, N,N-diphenylcarbamoyloxy, morpholinocarbonyloxy), an alkoxycarbonyloxy group (e.g., t-butyloxycarbonyloxy, iso-butyloxycarbonyloxy), an alkylcarbonyloxy group (e.g., t-butylcarbonyloxy), an alkyl group (e.g., methyl), an aryl group (e.g., phenyl), or a hydroxy group, with particular preference given to a hydrogen atom, a carbamoyloxy group, and an alkoxycarbonyloxy group.

There are no particular restrictions on the method for converting the triazoles of formula (III) to the pyrrolotriazoles of formula (X), and the conversion can be attained according to the methods described, for example, in the literatures mentioned above, JP-A No. 48376/1995, Japanese patent application No. 144077/1994, United States Patent No. 5,256,526, European Patent No. 628,867, and European patent application No. 95115061.4. The disclosures of these literatures are incorporated hereinto by reference.

Generally, it has been considered that it is very difficult to hydrazidize, selectively, only one of two ester groups of 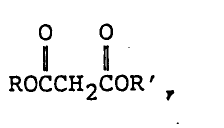 wherein R represents a primary alkyl group, with H₂NNH₂. Because this reaction resulted in a product composed of mixture of 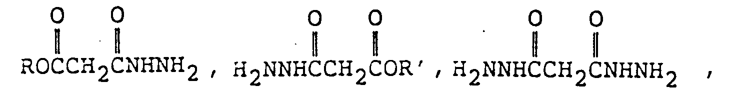 and the like. That is, since generally the compound, 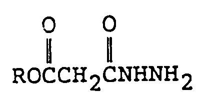 desired cannot be synthesized selectively and efficiently, it is not preferable to use this compound as a starting material. However, according to the present invention in the case that R represents 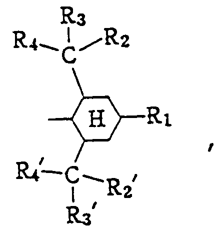 due to its steric hindrance, 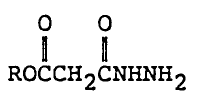 desired can be easily and efficiently synthedsized. Therefore this compound of formula (I) is possible to be used as the starting material.

The hydrazides (I) of the present invention are used as synthetic intermediates of IH-1,2,4-triazole derivatives (III), which are important intermediates in the synthesis of IH-pyrrolo-[1,2-b][l,2,4]triazole derivatives, which latter are useful as synthetic intermediates of physiologically active substances, such as medicines and pesticides, and which latter are also useful as photographic cyan couplers and dyes, particularly photographic cyan couplers. According to the method of the present invention, the above IH-1,2,4-triazole derivatives (III) can be produced efficiently in one step by using the above hydrazides (I).

### EXAMPLES

Now, the present invention is described in detail based on the following examples, which do not restrict the present invention.

### Example 1. Synthesis of Triazole (III-1a)

Triazole (III-1a) was synthesized in accordance with the following scheme:

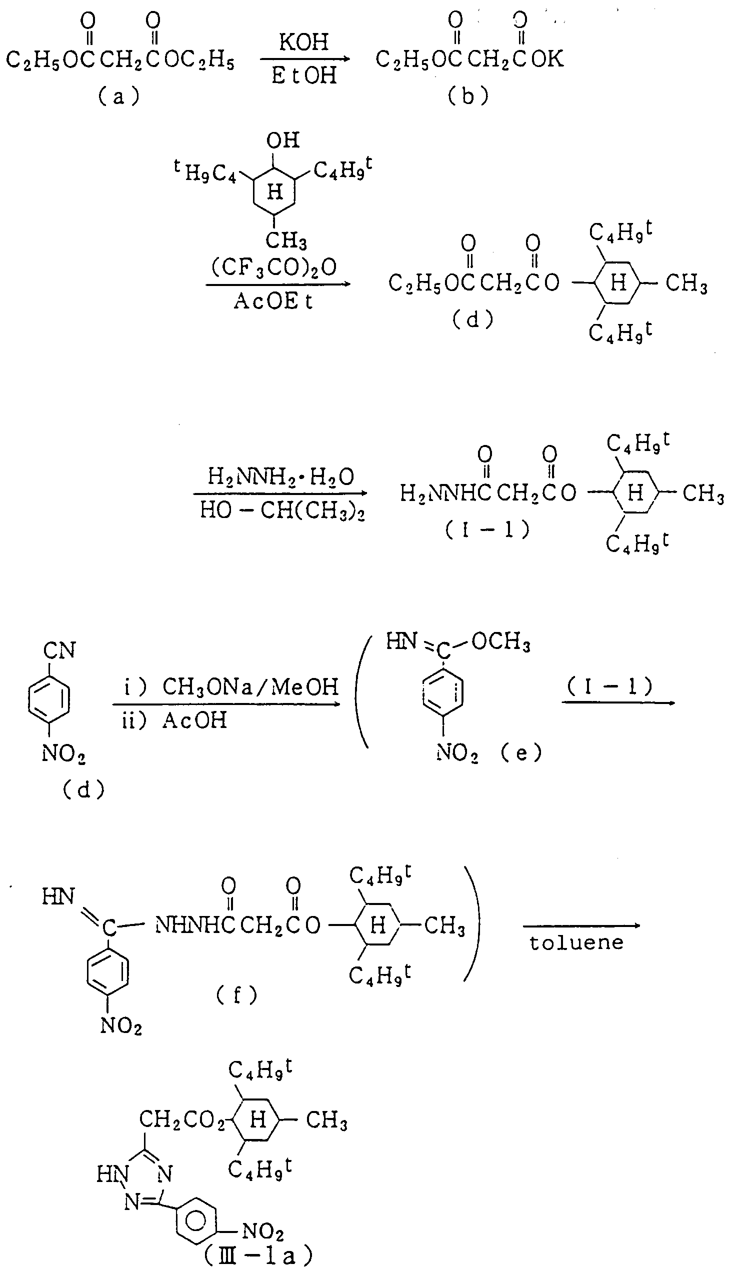

### Synthesis of malonic acid ethyl ester monopotassium salt (b)

A solution of potassium hydroxide (37.6 g, 0.67 mol) in ethanol (400 ml) was added, dropwise at room temperature, to a solution of diethyl maloate (108 g, 0.67 mol) in ethanol (300 ml). After the mixture was stirred at 50°C for 2 hours, it was cooled to 0°C. The deposited crystals were filtered and dried, to obtain crude malonic acid ethyl ester monopotassium salt (b) (87 g).

The crude malonic acid ethyl ester monopotassium salt (b) was directly used in the next step without purification.

### Synthesis of 2,6-di-tert-butyl-4-methylcyclohexanol

100 g of 2,6-di-tert-butylhydroxytoluene (BHT), 500 ml of isopropanol, and 10 g of Raney nickel catalyst were placed in a 1-liter stainless autoclave; the autoclave was sealed; replaced with hydrogen was carried out; and the mixture was stirred, under a hydrogen initial pressure of 100 kg/cm², for 9 hours at 100°C. After allowing it to stand, to cool to room temperature, the reaction product was taken out; the catalyst was filtered off, to be separated, and the solvent was distilled off, to obtain 2,6-di-tert-butyl-4-methylcyclohexanone (102 g).

8.6 g of aluminum lithium hydride was suspended in 200 ml of dry tetrahydrofuran, and the above 2,6-di-tert-butyl-4-methylcyclohexanone was added, dropwise, to the dispersion, over 20 min at 0°C with stirring.

The temperature of the reaction liquid was increased gradually to room temperature over 3 hours, and the reaction liquid was stirred at room temperature for a further 2 hours. Then 40 ml of methanol was added gradually thereto, to decompose the excess aluminum lithium hydride. After dilute hydrochloric acid was added, to make the reaction mixture acidic, 600 ml of ethyl acetate was added thereto, to carry out extraction, and the organic layer was separated. The organic layer was washed, successively, with dilute hydrochloric acid, an aqueous saturated sodium hydrogencarbonate solution, and brine, and then it was dried over anhydrous sodium sulfate.

The solvent was distilled off under reduced pressure, to obtain 101 g of crude 2,6-di-tert-butyl-4-methylcyclohexanol. The thus obtained crude 2,6-di-tert-butyl-4-methylcyclohexanol was used directly in the next step without purification.

### Synthesis of malonic acid ethyl 2,6-di-tert-butyl-4-methylcyclohexyl ester (d)

Trifluoroacetic anhydride (41 ml, 0.29 mol) was added, dropwise, to a solution of the above 2,6-di-tert-butyl-4-methylcyclohexanol (d) (50.2 g, 0.22 mol) in ethyl acetate (200 ml) at 0°C, and then the above malonic acid ethyl ester monopotassium salt (b) (41.6 g) was added thereto, slowly. After stirring it at room temperature for 2 hours, 200 ml of water was added thereto, and then 33 g of sodium hydrogencarbonate was added, slowly, to neutralize the reaction mixture. The organic layer was separated, washed with water once, and then with brine once; then it was dried over anhydrous sodium sulfate. The solvent was distilled off under reduced pressure, to obtain crude malonic acid ethyl 2,6-di-tert-butyl-4-methylcyclohexyl ester (d) (76 g). The thus obtained crude malonic acid ethyl 2,6-di-tert-butyl-4-methylcyclohexyl ester (d) was used directly in the next step without purification.

### Synthesis of Hydrazide (I-1)

A solution of the above malonic acid ethyl 2,6-di-tert-butyl-4-methylcyclohexyl ester (d) (73 g) and hydrazine monohydrate (28.3 g, 0.57 mol) in isopropanol (200 ml) was stirred for 6 hours, under reflux. 500 ml of water and 500 ml of ethyl acetate were added to the reaction liquid, to carry out extraction. The organic layer was washed with water twice, and then with brine twice; then it was dried over anhydrous sodium sulfate. The solvent was distilled off under reduced pressure, and the residue was recrystallized from hexane, to obtain Hydrazide (I-1) [53 g, 0.16 mol, 77% (based on 2,6-di-tert-butyl-4-methylcyclohexanol), and melting point: 105 to 106°C].

Incidentally, the structure was identified by NHR and mass spectrometry.

### Synthesis of Triazole (III-1a)

A 28% solution of sodium methylate (30 g, 0.155 mol) in methanol was added, dropwise at room temperature, to a solution of p-nitrobenzonitrile (d) (23 g, 0.155 mol) in methanol (120 ml). After they were stirred for 1 hour at room temperature, the mixture was allowed to stand for one day in a refrigerator (-5 to 0°C). After the reaction mixture was made neutral by adding, dropwise, acetic acid (19 g), at a temperature of 0°C or below (the procedure resulted in an imido ester (e)), the above Hydrazide (I-1) (53 g, 0.16 mol) was added thereto. After stirring the resulting mixture at room temperature for 3 hours (the procedure resulted in an intermediate (f)), the solvent was distilled off under reduced pressure. Toluene (400 ml) was added to the resulting concentrated residue, and the mixture was stirred, under reflux, for 3 hours, while removing produced water. After cooling the reaction mixture, 800 ml of each of ethyl acetate and water was added to the reaction mixture, followed by stirring for 30 min. The formed layers were separated, and the organic layer was washed with brine and dried over sodium sulfate. The solvent was distilled off under reduced pressure, followed by recrystallizing from ethyl acetate/hexane, to obtain the intended Triazole (III-1a) (60 g, 0.131 mol, 85%, and melting point: 168 to 170°C). Incidentally, the structure was identified by NMR and mass spectrometry.

### Example 2. Synthesis of Triazole (III-1b)

The method for producing Triazole (III-1a) in Example 1 was repeated, except that, instead of the p-nitrobenzonitrile (23 g), m-nitrobenzonitrile (23 g) was used, thereby obtaining Triazole (III-lb) (58 g, 1.27 mol, 82%, and melting point: 153 to 154°C).

Incidentally, the structure was identified by NMR and mass spectrometry.

### Example 3. Synthesis of Triazole (III-1q)

The synthesis of Triazole (III-1a) of Example 1 was repeated, except that, instead of the p-nitrobenzonitrile (23 g), 3-nitro-4-methylbenzonitrile (25 g) was used, thereby obtaining Triazole (III-1q) (45 g, 0.096 mol, 62%, and melting point: 178 to 180°C).

Incidentally, the structure was identified by NMR and mass spectrometry.

### Example 4. Synthesis of Triazole (III-1j)

Triazole (III-1j) was synthesized in accordance with the following scheme:

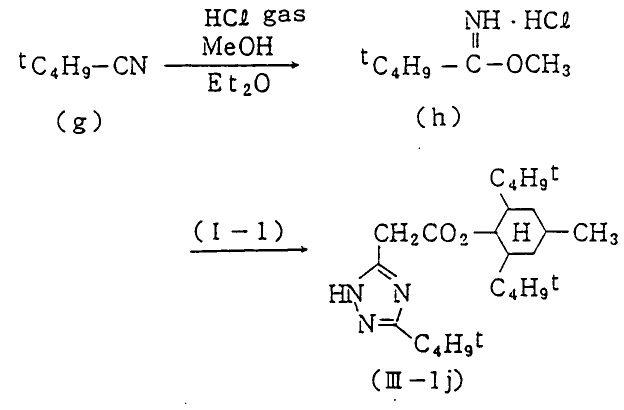

### Synthesis of an imido ester (h)

Hydrochloric acid gas (340 g, 9.32 mol) was blown into a solution of trimethylacetonitrile (g) (380 g, 4.57 mol) and methanol (161 g, 5.05 mol) in diethyl ether (750 ml), over 4 hours at 5°C. The reaction liquid was allowed to stand in a refrigerator (-5 to 0°C) for 1 week. The deposited crystals were filtered, washed with ether, and dried, to obtain an imido ester hydrochloride (h) (644 g, 4.25 mol, 93%).

### Synthesis of Triazole (III-1j)

A 28% solution of sodium methylate (25 g, 0.13 mol) in methanol was added, dropwise, to a solution of the above imido ester hydrochloride (h) (20 g, 0.13 mol) in isopropanol (100 ml), at room temperature, and then Hydrazide (I-1) (44.3 g, 0.13 mol) was added thereto. The reaction liquid was stirred, under reflux, for 5 hours. After the reaction liquid was cooled, 200 ml of each of ethyl acetate and water was added, to carry out extraction. After the organic layer was washed with water and then with brine, the organic layer was dried over sodium sulfate. After the solvent was distilled off under reduced pressure, the residue was recrystallized from hexane, to obtain the intended Triazole (III-1j) (33 g, 0.085 mol, 65%, and melting point: 138 to 139°C). Incidentally, the structure was identified by NMR and mass spectrometry.

The 1H-1,2,4-triazoles (III) obtained by using Hydrazides (I) of the present invention could be converted easily to 1H-pyrrolo-[1,2-b][1,2,4]triazole derivatives, which are useful as photographic cyan couplers, by the following method of the present invention.

### Example 5. Synthesis of 1H-pyrrolo-[1,2-b][1,2,4]triazoles

By using Triazole (III-1b) obtained above as a starting material, Compound Example (53) described in European Patent No. 628,827 was synthesized in accordance with the following scheme, based on a synthetic method described in JP-A No. 202004/1993.

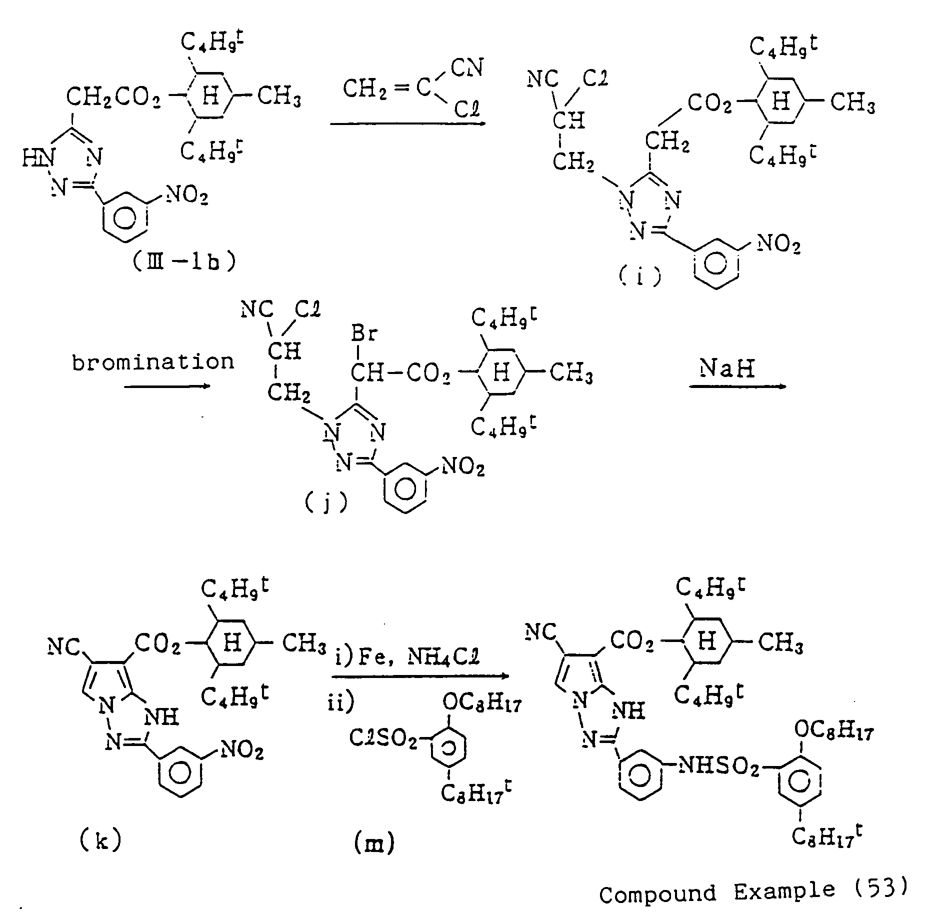

### Synthesis of a compound (k)

Compound (III-1b) (50.0 g, 109 mmol) and 2-chloroacrylonitrile (12.4 g, 142 mmol) were dissolved in 500 ml of tetrahydrofuran, and then 6.6 ml of tetramethylguanidine was added to the solution, at 0°C. After the resulting mixture was stirred at 0°C for 5 hours, 600 ml of ethyl acetate was added, and the ethyl acetate liquid was washed with water. The ethyl acetate layer was dried and distilled, and crystallization was carried out using methanol, to obtain 47.6 g (80%) of a compound (i).

The obtained compound (i) (47.6 g, 87.5 mmol) was dissolved in 370 ml of tetrahydrofuran, and then pyridinium bromide perbromide (33.5 g, 105 mmol) was added to the solution, under cooling with water. After the resulting mixture was stirred for 5 hours, 500 ml of ethyl acetate was added, followed by washing with water until the washings became neutral. After the ethyl acetate layer was dried and distilled, the residue was purified by column chromatography, to obtain 41.0 g (75%) of a compound (j).

The obtained compound (j) (41.0 g, 65.8 mmol) was dissolved in 200 ml of tetrahydrofuran, and, while keeping the reaction temperature at -10°C, 60% sodium hydride (10.5 g, 260 mmol) was added to the solution, little by little. After the reaction, ethyl acetate was added to the reaction mixture, followed by washing with water. The ethyl acetate layer was dried and distilled off, and the residue was purified by column chromatography, to obtain 5.0 g (yield: 15%) of a compound (k).

### Synthesis of Compound Example (53)

A mixture of the above-obtained compound (k) (5.0 g, 9.9 mmol), reduced iron (5 g), ammonium chloride (0.5 g), 10 ml of water, and 100 ml of isopropyl alcohol was stirred, under reflux, for 2 hours. The reaction mixture was Celite-filtered, and 200 ml of each of water and ethyl acetate was added to the filtrate. The organic layer was washed with brine and then dried over anhydrous sodium sulfate. After the solvent was distilled off under reduced pressure, the concentrated residue was added and dissolved in 50 ml of dimethylformamide. Pyridine (1.6 ml) and a sulfonyl chloride (m) (4.2 g) were added to the solution, and after the mixture was stirred for 6 hours at room temperature, 150 ml of an aqueous dilute hydrochloric acid solution and 150 ml of ethyl acetate were added thereto. After the organic layer was washed with water three times, the organic layer was dried over anhydrous sodium sulfate. After the solvent was distilled off under reduced pressure, the concentrated residue was recrystallized from ethyl acetate/hexane, to obtain Compound Example (53) (6.7 g, 79%) described in European Patent No. 628,827.

### Example 6. Synthesis of 1H-pyrrolo-[1,2-b][1,2,4]triazoles

By using Triazole (III-1q) as a starting material, 1H-pyrrolo-[1,2-b][1,2,4]triazoles (u) were synthesized in accordance with the following scheme:

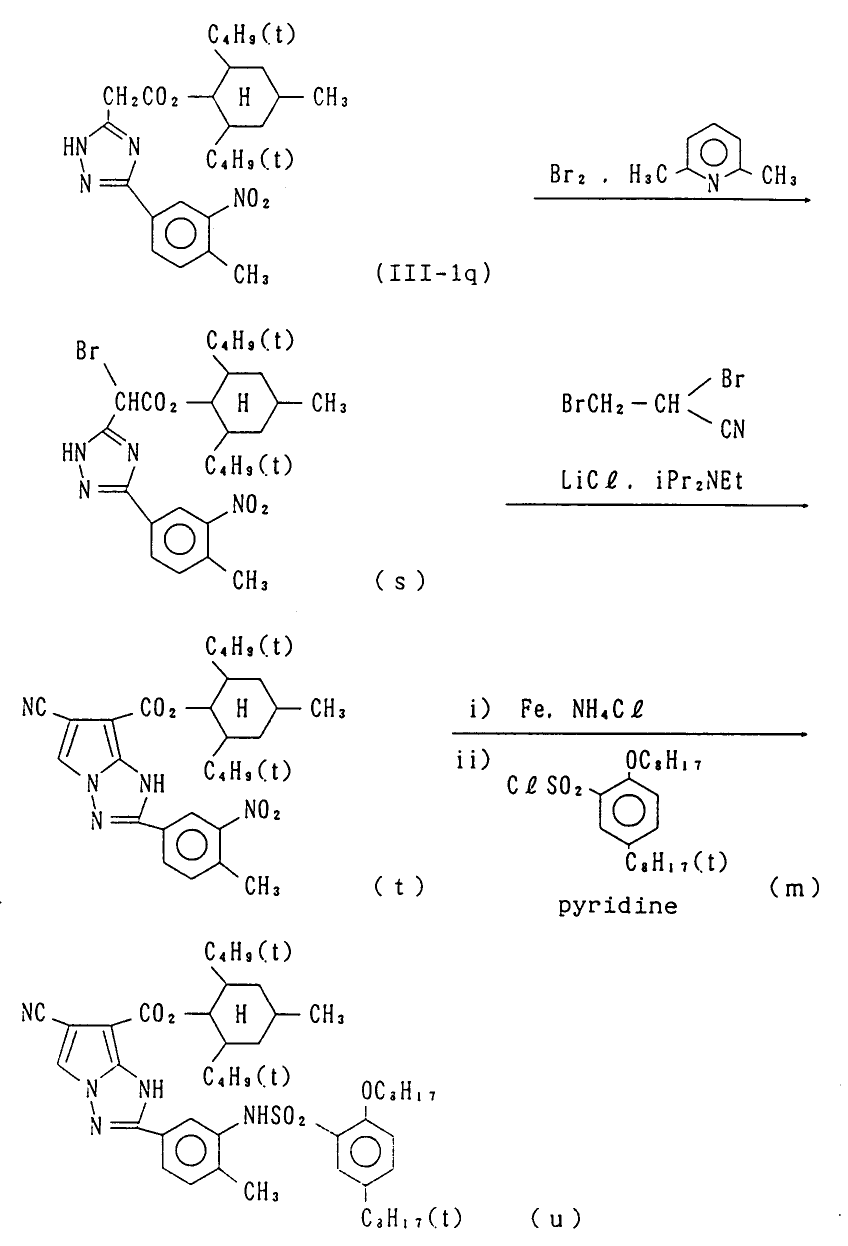

### Synthesis of a compound (s)

After bromine (7.30 g, 45.7 mmol) was added, dropwise, to a solution of Triazole (III-1q) (20.0 g, 42.5 mmol) and 2,6-lutidine (5.45 ml, 46.8 mmol) in ethyl acetate (200 ml), at room temperature, the mixture was stirred at 70°C for 30 min. After the reaction liquid was cooled to room temperature, 200 ml of 1N hydrochloric acid was added thereto. After the organic layer was washed with water and then with brine, the organic layer was dried over anhydrous sodium sulfate and concentrated. The concentrated residue was recrystallized from ethyl acetate/hexane, to obtain a compound (s) (19.6 g, 35.7 mmol, 83%).

### Synthesis of a compound (t)

A solution of lithium chrodide (38.5 g, 0.91 mol), the compound (s) (100 g, 0.182 mol), and 2,3-dibromopropionitrile (51.6 g, 0.242 mol) in dimethylformamide (1 liter) was cooled to 0°C or below, and then diisopropylethylamine (124 g, 0.96 mol) was added thereto, dropwise, with the temperature kept at 10°C or below. While the temperature of the reaction liquid was elevated gradually to room temperature, the reaction liquid was stirred for 2 hours. The reaction liquid was added to 1 liter of ethyl acetate and 1 liter of cool 1N hydrochloric acid, followed by stirring, and then the layers were separated. After the organic layer was washed with water and then with brine, the organic layer was dried over anhydrous sodium sulfate and concentrated. The concentrated residue was recrystallized from acetonitrile, to obtain a compound (t) (37 g, 71.2 mmol, 39%).

### Synthesis of a compound (u)

A mixture of the compound (t) (50 g, 96.2 mol), reduced iron (50 g), ammonium chloride (5 g), water (40 ml), isopropyl alcohol (400 ml), and tetrahydrofuran (100 ml) was stirred, under reflux, for 2 hours. The reaction mixture was Celite-filtered, and 750 ml of each of water and ethyl acetate was added to the filtrate. The organic layer was washed with brine; then it was dried over anhydrous sodium sulfate and concentrated. Pyridine (16 ml), a sulfonyl chloride (m) (42 g), and acetonitrile (500 ml) were added to the concentrated residue, and the mixture was stirred for 8 hours, under reflux. After the reaction liquid was cooled to room temperature, ethyl acetate (1.5 liters)/water (1.5 liters) was added thereto. After the organic layer was washed with water and then brine, the organic layer was dried over anhydrous sodium sulfate and concentrated. The concentrated residue was recrystallized from ethyl acetate/hexane, to obtain a compound (u) (61 g, 70 mmol, 73%).

Referring to JP-A No. 347960/1994, it can also be understood that a 1H-pyrrolo-[1,2-b][1,2,4]triazole derivative having an electron-attracting group (e.g., cyclohexyloxycarbonyl group) at the 7-position is a preferable pyrrolotriazole derivative. That is, when this pyrrolotriazole derivative is used for a photographic material, excellent heat and humidity fading resistance at low density part is attained.

### Comparative Example 1

Triazole (III-1j) was synthesized in accordance with the following scheme (conventional process):

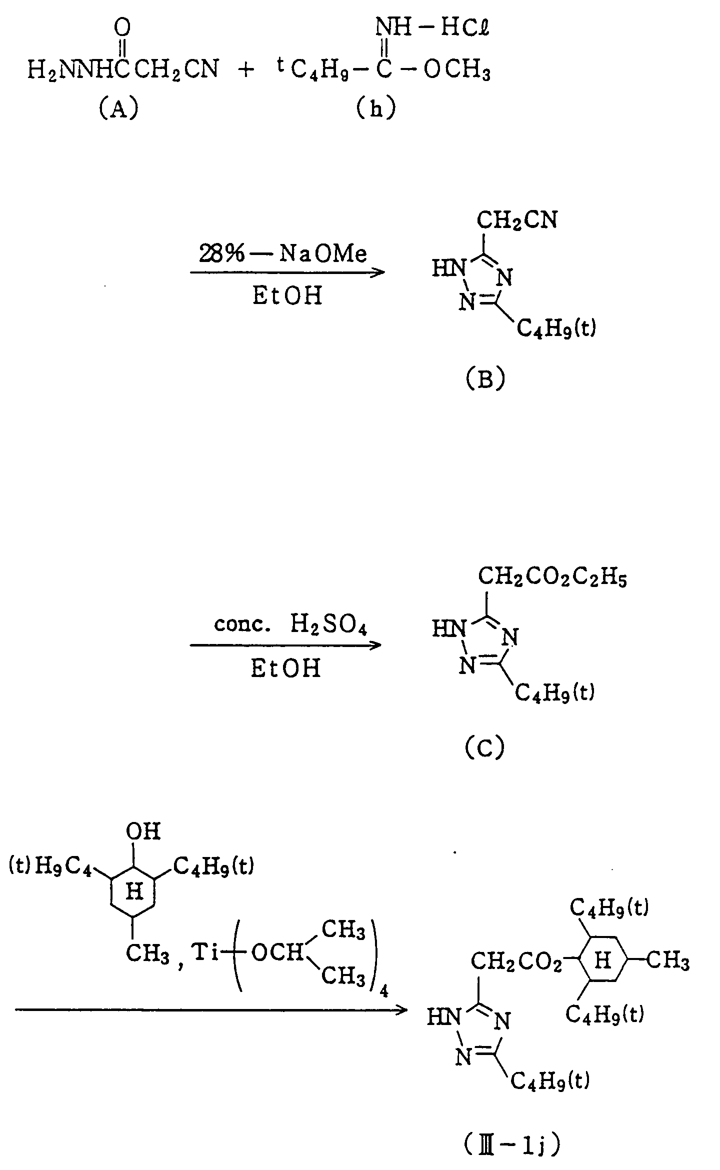

### Synthesis of Compound (III-1j)

A 28% solution of sodium methylate (19.7 g, 0.1 mol) in methanol was added, dropwise, at room temperature to a solution of cyanoacetohydrazide (A) (9.9 g, 0.1 mol) and imido ester hydrochloride (h) (15.2 g, 0.1 mol) in ethanol (160 ml). After the reaction liquid was stirred, under reflux, for 6 hours, the reaction liquid was concentrated under reduced pressure, to obtain a crude Compound (B) (containing table salt). (Since Compound (B) is soluble in water, without carrying out extraction, Compound (B) was used directly in the next step in the state containing inorganic substances.)

### Synthesis of Compound (C)

To the Compound (B) obtained above were added 60 ml of ethanol, 20 ml of concentrated sulfuric acid, and 1.2 ml of water, followed by stirring at 80°C for 5 hours. After the reaction liquid was cooled to room temperature, 160 ml of a 10% aqueous sodium hydroxide solution was added, slowly, thereto, and 200 ml of ethyl acetate was added, to carry out extraction. Then the organic layer was washed with water then with brine, dried over anhydrous sodium sulfate, and concentrated under reduced pressure. The concentrated residue was purified by column chromatography, to obtain an oil of Compound (C) (15 g, 0.071 mol; 71% in the two steps).

### Synthesis of Triazole (III-1j)

Compound (C) (15 g, 0.071 mol) obtained above, 2,6-di-t-butyl-4-methylcyclohexanol (46.5 g, 0.21 mol), and titanium tetraisopropoxide (0.5 mol) were stirred at 200°C for 1 hour under a nitrogen stream. After the reaction liquid was cooled to room temperature, the product was purified by column chromatography, to obtain Triazole (III-1j) (10 g, 0.026 mol; 36%).

By comparing Example 4 (Triazole (III-1j) was obtained in one step in a yield of 65% by using, as starting materials, Hydrazide I-1 and Imido Ester Hydrochloride (h)) with Comparative Example 1 (Triazole (III-1j) was obtained in three steps in a total yield of 26% by using, as starting materials, Cyanoacetohydrazide (A) and Imido Ester Hydrochloride (h)), it is apparent that in comparison with the conventional process, the production method of the present invention can synthesize the intended 1H-1,2,4-triazole derivative efficiently in short steps.

## Claims

1. A cyclohexyloxycarbonylacetohydrazide compound represented by formula (I): 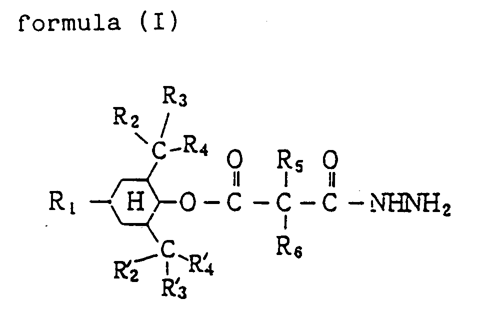 wherein R₁ represents a hydrogen atom or an alkyl group; R₂, R₃, R₄, R₂', R₃', and R₄', which are the same or different, each represent an alkyl group; R₂ and R₃, and R₂' and R₃' each may bond together to form a ring; and R₅ and R₆, which are the same or different, each represent a hydrogen atom, a halogen atom, an alkyl group, or an aryl group.

2. A method for producing 1H-1,2,4-triazole represented by formula (III): 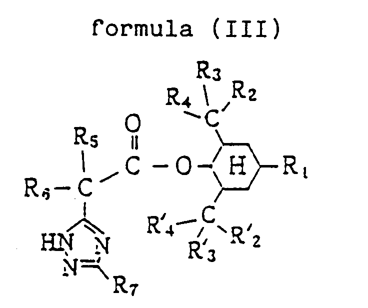 wherein R₁ represents a hydrogen atom or an alkyl group; R₂, R₃, R₄, R₂', R₃', and R₄', which are the same or different, each represent an alkyl group; R₂ and R₃, and R₂' and R₃' each may bond together to form a ring; R₅ and R₆, which are the same or different, each represent a hydrogen atom, a halogen atom, an alkyl group, or an aryl group; and R₇ represents an aliphatic group or an aryl group, comprising reacting a cyclohexyloxycarbonylacetohydrazide represented by formula (I): 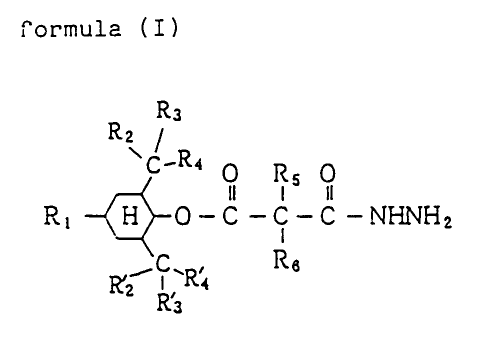 wherein R₁, R₂, R₃, R₄, R₂', R₃', R₄', R₅, and R₆ have the same meanings as defined above, with an imido ester represented by formula (II): 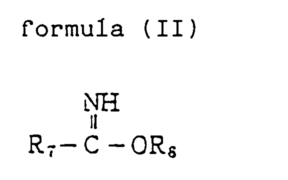 wherein R₇ has the same meaning as defined above; and R₈ represents a lower alkyl group, to give a 1H-1,2,4-triazole of formula (III).

3. The method as claimed in claim 2, wherein the cyclohexyloxycarbonylacetohydrazide represented by formula (I) is subjected to condensation reaction with the imido ester represented by formula (II), to give an intermediate represented by formula (IV): 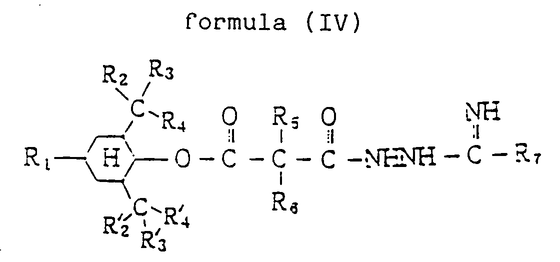 wherein R₁, R₂, R₃, R₄, R₂', R₃', R₄', R₅, R₆, and R₇ have the same meanings as defined above, and then the intermediate is subjected to intramolecular dehydrogenation condensation, to give a 1H-1,2,4-triazole represented by formula (III).

4. A method for producing 1H-pyrrolo-[1,2-b][1,2,4]triazole represented by formula (X): 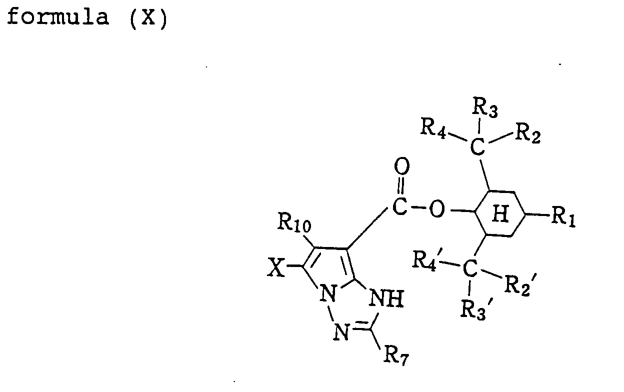 wherein R₁ represents a hydrogen atom or an alkyl group; R₂, R₃, R₄, R₂', R₃', and R₄', which are the same or different, each represent an alkyl group; R₂ and R₃, and R₂' and R₃' each may bond together to form a ring; R₇ represents an aliphatic group or an aryl group; R₁₀ represents a hydrogen atom or a substituent; and X represents a hydrogen atom or a substituent, from a 1H-1,2,4-triazole represented by formula (III): 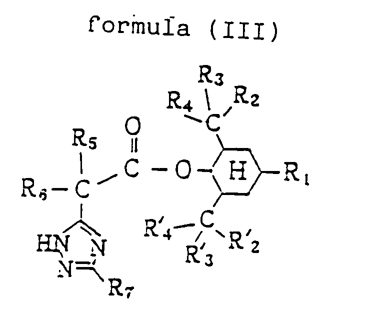 wherein R₁, R₂, R₃, R₄, R₂', R₃', R₄', and R₇ have the same meanings as defined above; R₅ and R₆, which are the same or different, each represent a hydrogen atom, a halogen atom, an alkyl group, or an aryl group, wherein the compound represented by formula (III) is prepared by reacting a cyclohexyloxycarbonylacetohydrazide represented by formula (I): 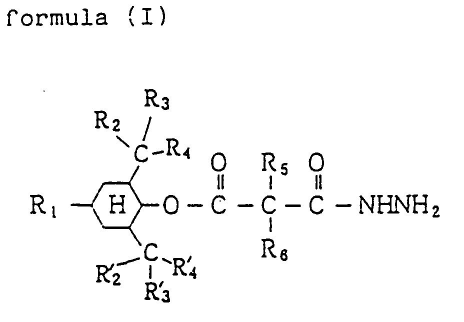 wherein R₁, R₂, R₃, R₄, R₂', R₃', R₄', R₅, and R₆ have the same meanings as defined above, with an imido ester represented by formula (II): 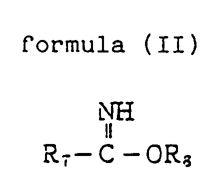 wherein R₇ has the same meaning as defined above; and R₈ represents a lower alkyl group.

5. The method as claimed in claim 4, wherein the cyclohexyloxycarbonylacetohydrazide represented by formula (I) is subjected to condensation reaction with the imido ester represented by formula (II), to give an intermediate represented by formula (IV): 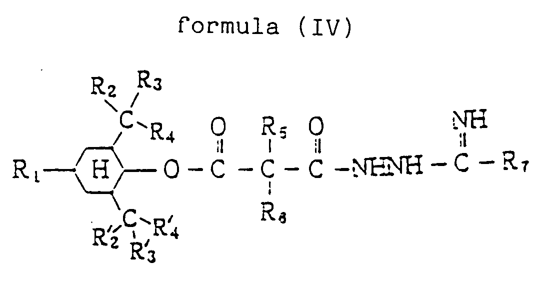 wherein R₁, R₂, R₃, R₄, R₂', R₃', R₄', R₅, R₆, and R₇ have the same meanings as defined above, and then the intermediate is subjected to intramolecular dehydrogenation condensation, to give a 1H-1,2,4-triazole represented by formula (III).

## Patentansprüche

1. Cyclohexyloxycarbonylacetohydrazidverbindung mit der Formel (I) 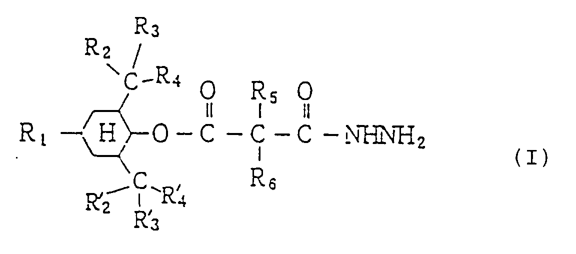 worin R₁ ein Wasserstoffatom oder eine Alkylgruppe bedeutet; R₂, R₃, R₄, R₂', R₃' und R₄', die gleich oder verschieden sind, jeweils eine Alkylgruppe bedeuten; R₂ und R₃ und R₂' und R₃' jeweils miteinander zur Bildung eines Ringes verbunden sein können; und R₅ und R₆, die gleich oder verschieden sind, jeweils ein Wasserstoffatom, ein Halogenatom, eine Alkylgruppe oder eine Arylgruppe bedeuten.

2. Verfahren zur Herstellung eines 1H-1,2,4-Triazols mit der Formel (III): 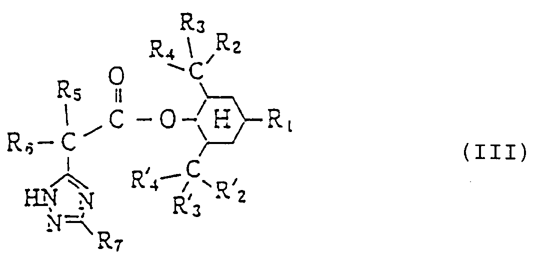 worin R₁ ein Wasserstoffatom oder eine Alkylgruppe bedeutet; R₂, R₃, R₄, R₂', R₃' und R₄', die gleich oder verschieden sind, jeweils eine Alkylgruppe bedeuten; R₂ und R₃ und R₂' und R₃' jeweils miteinander unter Bildung eines Ringes verbunden sein können; R₅ und R₆, die gleich oder verschieden sind, jeweils ein Wasserstoffatom, ein Halogenatom, eine Alkylgruppe oder eine Arylgruppe bedeuten; und R₇ eine aliphatische Gruppe oder eine Arylgruppe bedeutet, umfassend die Umsetzung eines Cyclohexyloxycarbonylacetohydrazids mit der Formel (I): 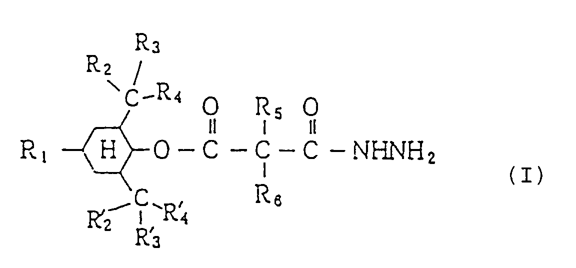 worin R₁, R₂, R₃, R₄, R₂', R₃', R₄', R₅ und R₆ die oben angegebenen Bedeutungen haben, mit einem Imidoester der Formel (II) 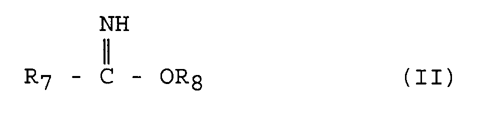 worin R₇ die oben angegebene Bedeutung hat; und R₈ eine Niederalkylgruppe darstellt, so dass ein 1H-1,2,4-Triazol der Formel (III) entsteht.

3. Verfahren gemäss Anspruch 2, worin das Cyclohexyloxycarbonylacetohydrazid mit der Formel (I) einer Kondensationsreaktion mit dem Imidoester der Formel (II) unterworfen wird, so dass ein Zwischenprodukt mit der Formel (IV) entsteht: 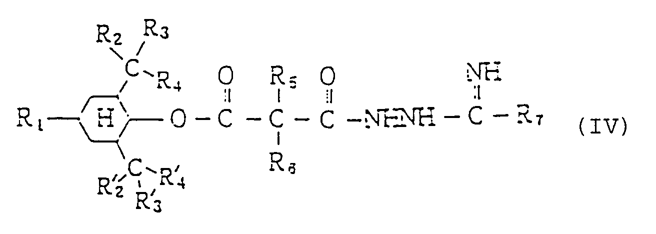 worin R₁, R₂, R₃, R₄, R₂', R₃', R₄', R₅, R₆ und R₇ die gleichen Bedeutungen wie oben haben und dann das Zwischenprodukt einer intramolekularen Dehydrierungskondensation unterworfen wird, so dass ein 1H-1,2,4-Triazol mit der Formel (III) entsteht.

4. Verfahren zur Herstellung von 1H-Pyrrolo[1,2-b][1,2,4]triazolen mit der Formel (X): 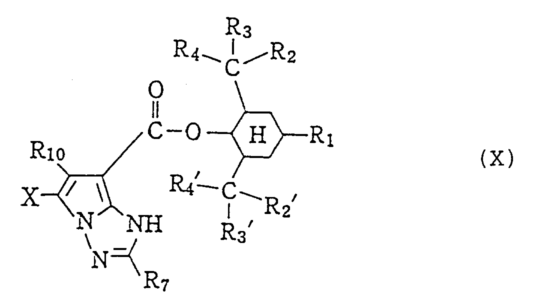 worin R₁ ein Wasserstoffatom oder eine Alkylgruppe bedeutet; R₂, R₃, R₄, R₂', R₃' und R₄', die gleich oder verschieden sind, jeweils eine Alkylgruppe bedeuten; R₂ und R₃ und R₂' und R₃' jeweils miteinander unter Bildung eines Ringes verbunden sein können; R₇ eine aliphatische Gruppe oder eine Arylgruppe bedeutet; R₁₀ ein Wasserstoffatom oder einen Substituenten bedeutet; und X ein Wasserstoffatom oder einen Substituenten bedeutet, aus einem 1H-1,2,4-Triazol mit der Formel (III): 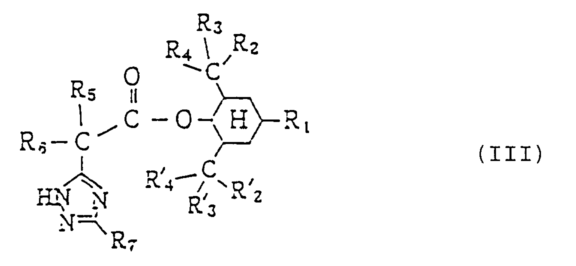 worin R₁, R₂, R₃, R₄, R₂', R₃', R₄' und R₇ die oben angegebenen Bedeutungen haben; R₅ und R₆, die gleich oder verschieden sind, jeweils ein Wasserstoffatom, ein Halogenatom, eine Alkylgruppe oder eine Arylgruppe bedeuten, worin die Verbindung mit der Formel (III) durch Umsetzung eines Cyclohexyloxycarbonylacetohydrazids mit der Formel (I): 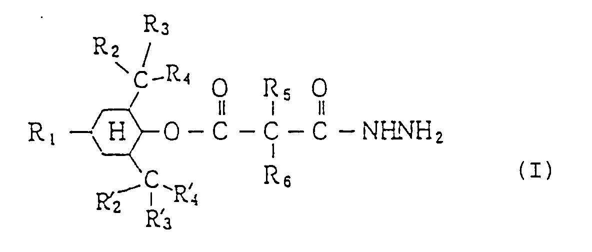 worin R₁, R₂, R₃, R₄, R₂', R₃', R₄', R₅ und R₆ die gleichen Bedeutungen wie oben haben, mit einem Imidoester der Formel (II): 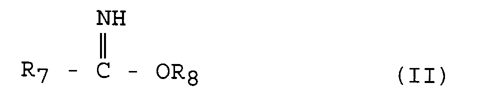 hergestellt wird, wobei R₇ die gleiche Bedeutung wie oben hat und R₈ eine Niederalkylgruppe bedeutet.

5. Verfahren gemäss Anspruch 4, worin das Cyclohexyloxycarbonylacetohydrazid mit der Formel (I) einer Kondensationsreaktion mit dem Imidoester der Formel (II) unterworfen wird, so dass ein Zwischenprodukt mit der Formel (IV) entsteht: 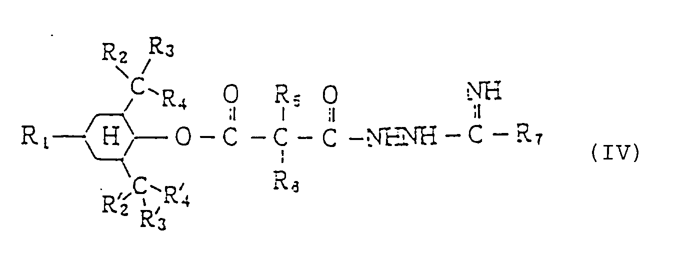 worin R₁, R₂, R₃, R₄, R₂', R₃', R₄', R₅, R₆ und R₇ die gleichen Bedeutungen wie oben haben, und dann das Zwischenprodukt einer intramolekularen Dehydrierungskondensation unterworfen wird, so dass ein 1H-1,2,4-Triazol mit der Formel (III) entsteht.

## Revendications

1. Composé de cyclohexyloxycarbonylacétohydrazide représénté par la formule (I): 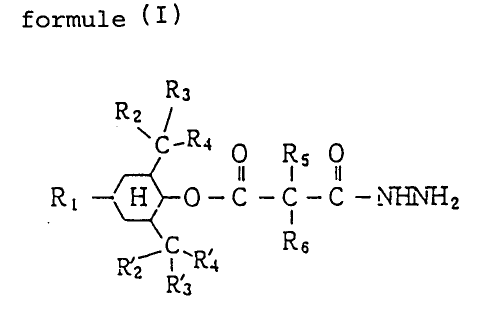 dans laquelle R₁ représente un atome d'hydrogène ou un groupe alkyle; R₂, R₃, R₄, R₂', R₃', et R₄', qui sont identiques ou différents, représentent chacun un groupe alkyle; R₂ et R₃, et R₂' et R₃' peuvent chacun se lier ensemble pour former un noyau; et R₅ et R₆, qui sont identiques ou différents, représentent chacun un atome d'hydrogène, un atome d'halogène, un groupe alkyle ou un groupe aryle.

2. Procédé de production d'un 1H-1,2,4-triazole représenté par la formule (III): 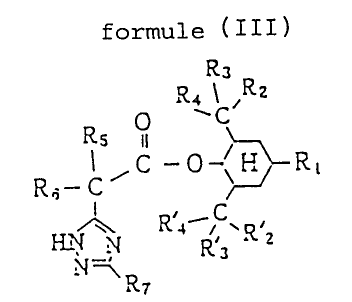 dans laquelle R₁ représente un atome d'hydrogène ou un groupe alkyle; R₂, R₃, R₄, R₂', R₃', et R₄', qui sont identiques ou différents, représentent chacun un groupe alkyle; R₂ et R₃, et R₂' et R₃' peuvent chacun se lier ensemble pour former un noyau; R₅ et R₆ qui sont identiques ou différents, représentent chacun un atome d'hydrogène, un atome d'halogène, un groupe alkyle ou un groupe aryle: et R₇ représente un groupe aliphatique ou un groupe aryle, consistant à faire réagir un cyclohexyloxycarbonylacétohydrazide représenté par la formule (I): 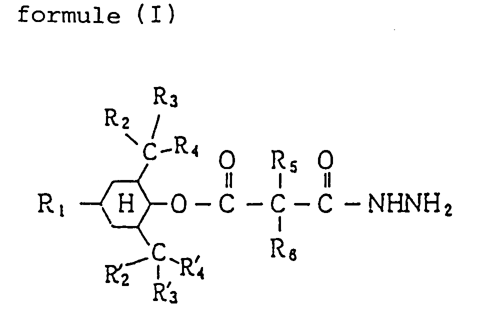 dans laquelle R₁, R₂, R₃, R₄, R₂', R₃', R₄', R₅, et R₆ ont les mêmes significations que celles données plus haut, avec un imido ester représenté par la formule (II): 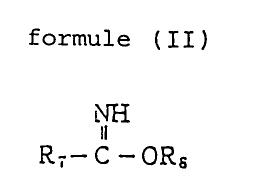 dans laquelle R₇ a la même signification que celle donnée plus haut; et R₈ représente un groupe alkyle inférieur, pour donner un 1H-1,2,4-triazole de formule (III).

3. Procédé selon la revendication 2, dans lequel le cyclohexyloxycarbonylacétohydrazide représenté par la formule (I) est soumis à une réaction de condensation avec l'imido ester représenté par la formule (II), pour donner un produit intermédiaire représenté par la formule (IV): 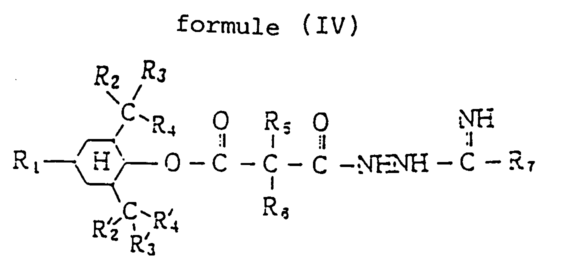 dans laquelle R₁, R₂, R₃, R₄, R₂', R₃', R₄', R₅, R₆, et R₇ ont les mêmes significations que celles données plus haut, et le produit intermédiaire est ensuite soumis à une condensation par déshydrogénation intramoléculaire, pour donner un 1H-l,2,4-triazole représenté par la formule (III).

4. Procédé de production de 1H-pyrrolo-[1,2-b]-[1,2,4]triazole représenté par la formule (X): 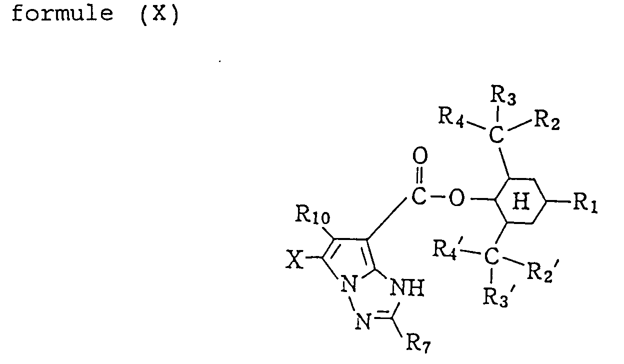 dans laquelle R₁ représente un atome d'hydrogène ou un groupe alkyle; R₂, R₃, R₄, R₂', R₃', et R₄' qui sont identiques ou différents, représentent chacun un groupe alkyle; R₂ et R₃, et R₂' et R₃' peuvent chacun se lier ensemble pour former un noyau; R₇ représente un groupe aliphatique ou un groupe aryle; R₁₀ représente un atome d'hydrogène ou un substituant; et X représente un atome d'hydrogène ou un substituant, à partir d'un 1H-1,2,4-triazole représenté par la formule (III): 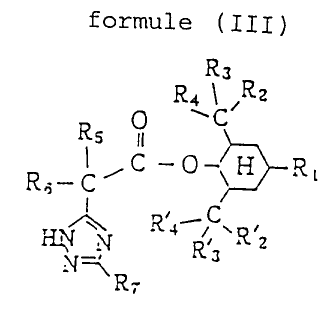 dans laquelle R₁, R₂, R₃, R₄, R₂', R₃,' R₄', et R₇ ont les mêmes significations que celles données plus haut; R₅ et R₆, qui sont identiques ou différents, représentent chacun un atome d'hydrogène, un atome d'halogène, un groupe alkyle ou un groupe aryle, dans lequel le composé représenté par la formule (III) est préparé en faisant réagir un cyclohexyloxycarbonylacétohydrazide représenté par la formule (I): 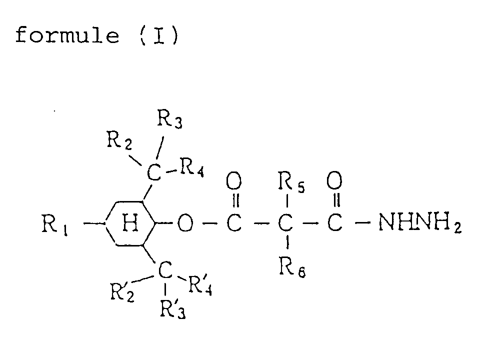 dans laquelle R₁, R₂, R₃, R₄, R₂', R₃', R₄', R₅ et R₆ ont les mêmes significations que celles données plus haut, avec un imido ester représenté par la formule (II): 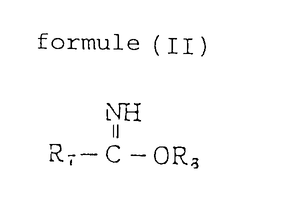 dans laquelle R₇ a la même signification que celle donnée ci-dessus, et R₈ représente un groupe alkyle inférieur.

5. Procédé selon la revendication 4, dans lequel le cyclohexyloxycarbonylacétohydrazide représenté par la formule (I) est soumis à une réaction de condensation avec l'imido ester représenté par la formule (II), pour donner un produit intermédiaire représenté par la formule (IV): 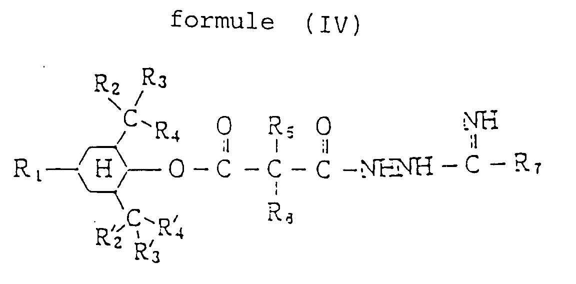 dans laquelle R₁, R₂, R₃, R₄, R₂', R₃', R₄', R₅, R₆, et R₇ ont les mêmes significations que celles données plus haut, et le produit intermédiaire est ensuite soumis à une condensation par déshydrogénation intramoléculaire, pour donner un 1H-1,2,4-triazole représenté par la formule (III).
